# EUROPEAN PATENT SPECIFICATION

(11) **EP 1 683 792 B1**
(45) Date of publication and mention of the grant of the patent: **20.07.2016**
(21) Application number: 04797287.2
(22) Date of filing: 04.11.2004
(51) Int. Cl.: C07D 311/16, A01N 43/16

(54) **BENZOPYRONE COMPOUNDS, PREPARATION METHOD AND USE THEREOF**
BENZOPYRONVERBINDUNGEN, HERSTELLUNGSVERFAHREN UND VERWENDUNG DAVON
COMPOSÉS DE BENZOPYRONE ET LEURS PROCÉDÉ DE PRÉPARATION ET D'UTILISATION

(30) Priority: 11.11.2003 CN 200310105079
(43) Date of publication of application: 26.07.2006
(73) Proprietor: Shenyang Sinochem Agrochemicals R & D Co., Ltd., Shenyang, Liaoning 110021 (CN)
(72) Inventor: LIU, Changling, Shenyang, Liaoning 110021 (CN); GUAN, Aiying, Shenyang, Liaoning 110021 (CN); ZHANG, Hong, Shenyang, Liaoning 110021 (CN); ZHANG, Mingxing, Shenyang, Liaoning 110021 (CN); LI, Zhengming, Tianjin 300071 (CN); LI, Miao, Shenyang, Liaoning 110021 (CN); LI, Lin, Shenyang, Liaoning 110021 (CN); LI, Zhinian, Shenyang, Liaoning 110021 (CN); HOU, Chunqing, Shenyang, Liaoning 110021 (CN)
(74) Representative: Bailey, Sam Rogerson
(86) International application number: PCT/CN2004/001255
(87) International publication number: WO 2005/044813

(56) References cited:
- WO-A-2005/123054
- WO-A1-01/98288
- JP-B2- 2 897 789
- US-A- 4 372 970
- DATABASE CA [Online] CHEMICAL ABSTRACTS SERVICE, COLUMBUS, OHIO, US; HAYASE, YOSHIO ET AL: "Preparation of (alkoxyimino)benzeneacetamide derivatives as agrochemical fungicides" XP002494465 retrieved from STN Database accession no. 1993:59429 & JP 04 182461 A (SHIONOGI AND CO., LTD., JAPAN) 30 June 1992 (1992-06-30)

## Description

### FIELD OF THE INVENTION

The invention relates to insecticides and fungicides, specifically to benzopyrone compounds and its preparation method and use thereof.

### BACKGROUND OF THE INVENTION

Biologically active natural products include benzopyrone and strobilurin (methoxyacrylate) compounds. Compounds of the following general formula have ever been published in JP04-182461:

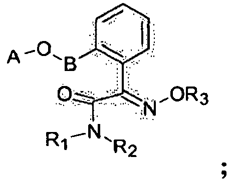

The structure of compound **JP51** in JP04-182461 is as follows:

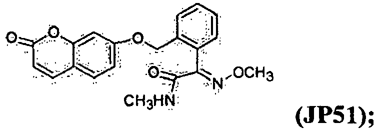

Biological active data of the compound in JP04-182461 have not been disclosed. After synthesis and the biological evaluation, it was found that compound **JP51** has low biological activity.

WO 01/98288 discloses phenyl-substituted 5,6-dihydropyrone derivatives for use as pesticides (especially insecticides) and herbicides. Typical compounds have the formula:

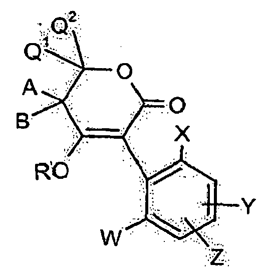

Where RO may be an ester residue.

### SUMMARY OF THE INVENTION

The aim of the present invention is to provide benzopyrone compounds with biological activity against all sorts of plant diseases and insects at very low dosage, and the compounds can be applied in agriculture to control diseases and insects in plant.

Detailed description of the invention is as follows:

The present invention provides benzopyrone compounds having general formula (I): 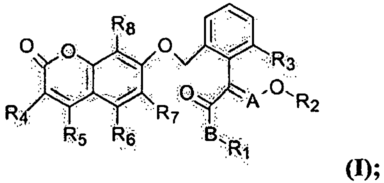 and stereoisomers thereof,
wherein: A is CH or N;
B is O or S;
R₁ and R₂ are respectively selected from H, C₁-C₁₂alkyl or C₁-C₁₂ haloalkyl;
R₃ is selected from H, C₁-C₁₂alkyl, C₁-C₁₂ haloalkyl or C₁-C₁₂ alkoxy;
R₄, R₅, R₆, R₇, and R₈ may be the same or different, and are selected from H, halo, CN; NO_{2;} C₁-C₁₂alkyl; C₂-C₁₂alkenyl; C₂-C₁₂alkynyl; C₁-C₁₂haloalkyl; C₁-C₁₂alkoxy; C₁-C₁₂alkylthio; C₁-C₁₂alkylsulfonyl; C₁-C₁₂alkylcarbonyl; C₁-C₁₂alkoxyC₁-C₁₂alkyl; C₁-C₁₂alkoxycarbonyl; C₁-C₁₂alkoxycarbonyl C₁-C₁₂alkyl; C₁-C₁₂haloalkoxyC₁-C₁₂alkyl; amino C₁-C₁₂alkyl in which amino is substituted with 0-2 C₁-C₁₂ alkyl; optionally substituted aryl, aryloxyl, arylC₁-C₁₂alkyl, arylC₁-C₁₂alkoxy, aryloxyC₁-C₁₂zalkyl, arylC₁-C₁₂alkoxylC₁-C₁₂alkyl, heteroaryl, heteroarylC₁-C₁₂alkyl, or heteroarylC₁-C₁₂alkoxyl groups, said optional substituents being up to 3 groups selected from (a) halo, (b) NO₂, (c) C₁-C₆alkyl, (d) C₁-C₆haloalkyl, (e) C₁-C₆alkoxy and (f) C₁-C₆alkoxyC₁-C₆alkyl; and groups having general formula: 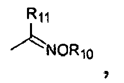 wherein: R₁₀ and R₁₁ are selected from (a) H, (b) C₁-C₁₂ alkyl, (c) aryl and (d) aryl C₁-C₁₂alkyl.

The preferred compounds of general formula **(I)** of this invention are:
A is CH or N;
Bis O or S,
R₁ and R₂ are respectively selected from H, C₁-C₆alkyl or C₁-C₆ haloalkyl;
R₃ is selected from H, C₁-C₆alkyl, C₁-C₆ haloalkyl or C₁-C₆alkoxy;
R₄, R₅, R₆, R₇, and R₈ may be the same or different, selected from H, halo; CN; NO_{2;} C₁-C₆alkyl; C₂-C₆alkenyl; C₂-C₆alkynyl; C₁-C₆haloalkyl; C₁-C₆alkoxy; C₁-C₆ alkylthio; C₁-C₆alkylsulfonyl; C₁-C₆alkylcarbonyl; C₁-C₆ alkoxyC₁-C₆alkyl; C₁-C₆alkoxycarbonyl; C₁-C₆alkoxycarbonylC₁-C₆alkyl; C₁-C₆haloalkoxyC₁-C₆alkyl; amino C₁-C₆alkyl in which amino is optionally substituted with up to 2 C₁-C₁₂ alkyl; optionally substituted aryl, aryloxyl, arylC₁-C₆alkyl, arylC₁-C₆alkoxy, aryloxyC₁-C₆alkyl, arylC₁-C₆alkoxylC₁-C₆alkyl, heteroaryl, heteroarylC₁-C₆alkyl, or heteroarylC₁-C₆alkoxyl groups, said optional substituents being up to 3 groups selected from halo, NO₂, C₁-C₂alkyl, C₁-C₂haloalkyl, C₁-C₂alkoxy or C₁-C₂alkoxyC₁-C₂alkyl, and groups having formula are as follows: 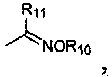 wherein: R₁₀ and R₁₁ are respectively selected from H, C₁-C₁₂alkyl, aryl or arylC₁-C6alkyl.

Still more preferred are such compounds wherein:
B is O;
R₁ and R₂ are both methyl;
R₃ is H or methyl;
R₄, R₅, R₆, R₇, and R₈ may be the same or different, and are selected from H, halo, CN, NO₂, C₁-C₆alkyl, C₂-C₆ alkenyl, C₁-C₆haloalkyl, C₁-C₆alkoxy, C₁-C₆alkylcarbonyl, C₁-C₆ alkoxyC₁-C₆alkyl, C₁-C₆alkoxycarbonyl, C₁-C₆alkoxycarbonylC₁-C₃alkyl, C₁-C₃haloalkoxyC₁-C₃alkyl, or amino C₁-C₃alkyl in which amino is optionally substituted with up to 2 C₁-C₃ alkyl; optionally substituted phenyl, phenoxy, phenylC₁-C₂alkyl, phenylC₁-C₂alkoxy, phenoxyC₁-C₂alkyl, phenylmethyl, phenylmethoxyl, or phenylmethoxyC₁-C₂alkyl groups, said substituents being up to 2 groups selected from halo, NO₂, C₁-C₂alkyl, C₁-C₂haloalkyl, C₁-C₂alkoxy or C₁-C₂alkoxyC₁-C₂alkyl, and groups having general formula as follows: 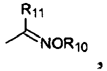 wherein: R₁₀ and R₁₁ are respectively selected from H and C₁-C₆alkyl.

Still more preferably,
R₄, R₅, R₆, R₇, and R₈ may be the same or different, respectively selected from H, Cl, Br, F, CN, C₁-C₆alkyl, C₁-C₆haloalkyl, C₁-C₆alkylcarbonyl, C₁-C₆alkoxy, C₁-C₆alkoxyC₁-C₃alkyl, C₁-C₃haloalkoxyC₁-C₃alkyl, amino C₁-C₃alkyl in which amino is optionally substituted with up to 2 C₁-C₃ alkyl; optionally substituted phenyl, phenoxy, phenylmethyl, phenylmethoxyl groups, said substituents being up to 2 groups selected from: halo, NO₂, C₁-C₂alkyl, C₁-C₂haloalkyl, C₁-C₂alkoxy or C₁-C₂alkoxyC₁-C₂alkyl, and groups having general formula as follows: 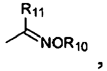 wherein: R₁₀ and R₁₁ are both methyl.

The following is the meaning of terms in the general formula (I):
Halogen or halo is meant to include fluoro, chloro, bromo or iodo.

The term alkyl includes both straight and branched chain alkyl such as methyl, ethyl, propyl, isopropyl and tert-butyl.

The term haloalkyl refers to straight or branched chain alkyl , in which hydrogen atom may be all or partly substituted with halogen, such as chloromethyl, dichloromethyl, trichloromethyl, fluoromethyl, difluoromethyl, or trifluoromethyl.

The term alkoxy refers to straight or branched chain alkyl, which is linked to the structure by oxygen atom.

The term haloalkoxy refers to straight or branched chain alkoxy, in which hydrogen atom may be all or partly substituted with halogen-, such as chloromethoxy, dichloromethoxy, trichloromethoxy, fluoromethoxy, difluoromethoxy, trifluoromethoxy, chlorofluoromethoxy, or trifluoroethoxy.

The term alkenyl refers to an straight or branched, having double bonds at any position such as vinyl or allyl. Substituted alkenyl includes arylvinyl substituted at any position with any group.

The term alkynyl refers to straight or branched groups having triple bonds at any position, such as ethynyl or propynyl. Substituted alkynyl includes arylethynyl substituted at any position with any group.

The terms aryl and aryl in arylalkyl, arylalkenyl arylalkynyl, aryloxy and aryloxyalkyl include phenyl and naphthyl.

The substituent groups in phenyl, phenoxy, phenylmethyl and phenylmethoxy are such groups as alkyl, alkoxy, haloalkyl, haloalkoxy, halo, NO₂ and CN. The number of the substituent groups can be from one to five.

The term heteroaryl in this invention refers to five member ring or six member ring containing one or many N, O, S hetero atom such as furan, thiophene, pyrrole, pyrazole, imidazole, thiazole, triazole, pyridine, pyrimidine, pyrazine, pyridazine, triazine, quinoline, benzofuran.

Because of the C=C and C=N link to different substituted group, the compounds of the invention may form geometrical isomer (the different isomers are respectively expressed with Z and E). Z isomer and E isomer and their mixture in any proportion are included in the invention.

The present invention is explained by the compounds of the following table I, but without being restricted thereby. 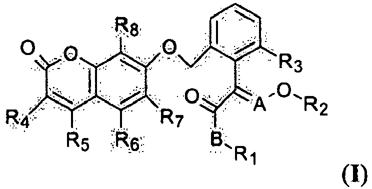 Wherein R₁, R₂=CH₃;E is C(CH₃)=NOCH₃; M is C₆H₃-3, 4-(OCH₃)₂

**Table 1**

| **No**. | **A** | **B** | **R₃** | **R₄** | **R₅** | **R₆** | **R₇** | **R₈** | **Physical-property *** |
|---|---|---|---|---|---|---|---|---|---|
| 1 | CH | O | H | H | H | H | H | H | oil |
| 2 | CH | 0 | H | H | CH₃ | H | H | H | 140-143 |
| 3 | CH | O | H | H | CH₃ | H | H | CH₃ | 188-190 |
| 4 | CH | O | H | H | C₆H₅ | H | H | CH₃ | 146-148 |
| 5 | CH | O | H | CH₃ | CH₃ | H | H | H | 120-122 |
| 6 | CH | O | H | CH₃ | CH₃ | H | H | CH₃ | 174-176 |
| 7 | CH | O | H | H | CF₃ | H | H | H | 164-166 |
| 8 | CH | O | H | H | CH₃ | H | H | E | oil |
| 9 | CH | O | H | H | CH₃ | H | E | H | 83-185 |
| 10 | CH | 0 | H | H | CH₃ | H | COCH₃ | H | 169-172 |
| 11 | CH | O | H | H | CH₃ | H | H | COCH₃ | 165-167 |
| 12 | CH | O | H | Cl | CH₃ | H | H | H | 162-164 |
| 13 | CH | O | H | H | CH₂Cl | H | H | H | |
| 14 | CH | O | H | Cl | CH₂Cl | H | H | H | |
| 15 | CH | O | H | Cl | CH₂OCH₃ | H | H | H | |
| 16 | CH | O | H | Cl | CH₂CH₃ | H | H | H | |
| 17 | CH | O | H | H | CH₂CH₃ | H | H | CH₃ | 154-156 |
| 18 | CH | O | H | C₂H₅ | CH₃ | H | H | H | 132-135 |
| 19 | CH | O | H | H | CH₂OCH₃ | H | H | H | 140-142 |
| 20 | CH | O | H | H | CH₂OC₂H₅ | H | H | H | |
| 21 | CH | O | H | Cl | CH₂OC₂H₅ | H | H | H | |
| 22 | CH | O | H | OCH₃ | CH₂OCH₃ | H | H | H | |
| 23 | CM | O | H | N(CH₃)₂ | CH₃ | H | H | H | |
| 24 | CH | O | H | CN | H | H | H | H | 166-168 |
| 25 | CH | O | H | Cl | CH₃ | H | H | CH₃ | 202-204 |
| 26 | CH | O | H | H | CH(CH₃)₂ | H | H | H | 128-130 |
| 27 | CH | O | H | C₃H₇ | CH₃ | H | H | H | 142-144 |
| 28 | CH | O | H | H | t C₄H₉ | H | H | H | |
| 29 | CH | O | H | H | 4-Cl-C₆H₄ | H | H | H | 149-152 |
| 30 | CH | O | H | Cl | 4-Cl-C₆H₄ | H | H | H | |
| 31 | CH | O | H | H | 4-Cl-C₆H₄ | H | H | CH₃ | |
| 32 | CH | O | H | Cl | C₆H₅ | H | H | H | 142-144 |
| 33 | CH | O | H | H | CH₂CH₃ | H | H | H | 134-136 |
| 34 | CH | O | H | H | CH₂C₂H₅ | H | H | H | 118-120 |
| 35 | CH | O | H | H | CH₂C₂H₅ | H | H | CH₃ | 146-148 |
| 36 | CH | O | H | Cl | CH₂C₂H₅ | H | H | H | 118-120 |
| 37 | CH | O | H | CH₃ | CH₂C₂Hs | H | H | H | 112-115 |
| 38 | CH | O | H | H | 4-F-C₆H₄ | H | H | H | 132-134 |
| 39 | CH | O | H | Cl | 4-F-C₆H₄ | H | H | H | |
| 40 | CH | O | H | H | 4-F-C₆H₄ | H | H | CH₃ | |
| 41 | CH | 0 | H | H | 4-CF₃-C₆H₄ | H | H | H | 161-162 |
| 42 | CH | O | H | Cl | 4-CF₃-C₆H₄ | H | H | H | |
| 43 | CH | O | H | Cl | CH₂N(CH₃)₂ | H | H | H | |
| 44 | CH | O | H | OCH₃ | C₂H₅ | H | H | H | |
| 45 | CH | O | H | OCH₃ | CH₃ | H | H | H | |
| 46 | CH | O | H | OC₂H₅ | CH₃ | H | H | H | |
| 47 | CH | O | H | H | CH₂OCH₂CF₃ | H | H | H | |
| 48 | CH | O | H | Cl | CH₂OCH₂CF₃ | H | H | H | |
| 49 | CH | O | H | F | CF₃ | H | H | H | |
| 50 | CH | O | H | F | CH₃ | H | H | H | 163-164 |
| 51 | CH | O | H | H | CH₂N(CH₃)₂ | H | H | H | |
| 52 | CH | O | H | H | C₆H₅ | H | H | H | 130-133 |
| 53 | CH | O | H | Cl | Cl | H | H | H | |
| 54 | CH | O | H | F | Cl | H | H | H | |
| 55 | CH | O | H | H | CH₂OCH₂C₆H₅ | H | E | H | |
| 56 | CH | O | H | OCH3 | 4-Cl-C₆H₅ | H | H | H | |
| 57 | CH | O | H | F | 4-Cl-C₆H₅ | H | H | H | |
| 58 | CH | O | H | H | M | H | H | H | 81-83 |
| 59 | CH | O | H | Cl | M | H | H | H | |
| 60 | CH | O | H | Cl | M | H | H | CH₃ | |
| 61 | CH | O | H | CH₃S | CH₃ | H | H | H | |
| 62 | CH | O | H | CH₃SO₂ | CH₃ | H | H | H | |
| 63 | CH | O | H | F | F | H | H | H | |
| 64 | CH | O | H | CH₃SO₂ | Cl | H | H | H | |
| 65 | CH | O | H | H | 4-NO₂-C₆H₅ | H | H | H | |
| 66 | CH | O | H | Cl | 4-NO₂-C₆H₅ | H | H | H | |
| 67 | CH | O | H | H | 4-NO₂-C₆H₅ | H | H | CH₃ | |
| 68 | CH | O | H | PhCH₂ | CH₃ | H | H | H | 159-162 |
| 69 | CH | O | H | PhCH₂ | CH₃ | H | H | CH₃ | |
| 70 | CH | O | H | CF₃CH₂O | C₃H₇ | H | H | H | |
| 71 | N | O | H | Cl | CH₃ | H | H | H | 172-174 |
| 72 | N | O | H | H | CH₃ | H | H | H | 150-152 |
| 73 | N | O | H | H | CH₃ | H | H | CH₃ | 178-180 |
| 74 | N | O | H | CH₃ | CH₃ | H | H | H | 112-118 |
| 75 | N | O | H | F | CH₃ | H | H | H | |
| 76 | N | O | H | H | CF₃ | H | H | Cl | |
| 77 | N | O | H | CH₃ | CH₃ | H | H | CH₃ | 184-186 |
| 78 | N | O | H | H | CH₃ | H | E | CO₂CH₃ | |
| 79 | N | O | H | H | CH₃ | H | COCH₃ | CO₂CH₃ | |
| 80 | N | O | H | Cl | CH₃ | H | H | CH₃ | 198-200 |
| 81 | N | O | H | H | CH₂Cl | H | H | CO₂CH₃ | |
| 82 | N | O | H | H | H | H | H | H | 106-110 |
| 83 | N | O | H | H | CH₂Cl | H | H | CF₃ | |
| 84 | N | O | H | H | 3-CF₃-C₆H₄ | H | H | CF₃ | |
| 85 | N | O | H | CH₃ | 3-CH₃-C₆H₄ | H | H | CF₃ | |
| 86 | N | O | H | CH₃ | 4-CH₃-C₆H₄ | H | H | CF₃ | |
| 87 | N | O | H | H | CH₂Cl | H | H | H | |
| 88 | N | O | H | Cl | CH₂Cl | H | H | H | |
| 89 | N | O | H | Cl | CH₂F | H | H | H | |
| 90 | N | O | H | H | CH₂F | H | H | H | |
| 91 | N | O | H | H | CH₂Br | H | H | H | |
| 92 | N | O | H | H | CH₂OCH₃ | H | H | CH₂N(CH₃)₂ | |
| 93 | N | O | H | Cl | CH₂OCH₃ | H | H | CH₂N(CH₃)₂ | |
| 94 | N | O | H | CH₃ | CH₂OCH₃ | H | H | CH₂N(CH₃)₂ | |
| 95 | N | O | H | H | CH₂OCH₃ | H | H | F | |
| 96 | N | O | H | CH₃ | CH₂OCH₃ | H | H | F | |
| 97 | N | O | H | CH₃ | CH₂OCH₃ | H | CO₂CH₃ | CH₂N(CH₃)₂ | |
| 98 | N | O | H | H | CH₂OCH₃ | H | H | H | |
| 99 | N | O | H | H | CH₂OCH₃ | H | H | E | |
| 100 | N | O | H | H | 3-CF₃-C₆H₄ | H | E | H | |
| 101 | N | O | H | H | 3-CH₃-C₆H₄ | H | COCH₃ | H | |
| 102 | N | O | H | H | 4-CH₃-C₆H₄ | H | H | COCH₃ | |
| 103 | N | O | H | Cl | CH₂OC₂H₅ | H | H | H | |
| 104 | N | O | H | H | CH₂OC₂H₅ | H | H | H | |
| 105 | N | O | H | H | CH₂OC₂Hs | H | H | CH₃ | |
| 106 | N | O | H | H | 3-OCH₃-C₆H₄ | H | H | CH₃ | |
| 107 | N | O | H | CH₃ | 4-OCH₃-C₆H₄ | H | H | H | |
| 108 | N | O | H | CH3 | 2-OCH₃-C₆H₄ | H | H | CH₃ | |
| 109 | N | O | H | H | CH₂OC₂H₅ | H | H | Cl | |
| 110 | N | O | H | H | CH₂OC₂H₅ | H | H | E | |
| 111 | N | O | H | H | M | H | E | H | |
| 112 | N | O | H | H | 3-CF₃-C₆H₄ | H | COCH₃ | H | |
| 113 | N | O | H | H | 3-CH₃-C₆H₄ | H | H | COCH₃ | |
| 114 | N | O | H | H | 4-CH₃-C₆H₄ | H | H | H | |
| 115 | N | O | H | H | 2-Cl-C₆H₄ | H | H | H | |
| 116 | N | O | H | H | 3-Cl-C₆H₄ | H | H | CH₃ | |
| 117 | N | O | H | H | CH₂OCH₂CF₃ | H | H | CH₃ | |
| 118 | N | O | H | CH₃ | CH₂OCH₂CF₃ | H | H | H | |
| 119 | N | O | H | CH₃ | -CH₂OC₆H₅ | H | H | CH₃ | |
| 120 | N | O | H | H | -CH₂OC₆H₅ | H | H | H | |
| 121 | N | O | H | H | CH₂OCH₂C₆H₅ | H | H | E | |
| 122 | N | 0 | H | H | CH₂OCH₂C₆H₅ | H | E | H | |
| 123 | N | O | H | H | 4-Cl-C₆H₄ | H | COCH₃ | H | |
| 124 | CH | O | CH₃ | H | H | H | H | H | |
| 125 | CH | O | CH₃ | H | CH₃ | H | H | H | |
| 126 | CH | O | CH₃ | H | CH₃ | H | H | CH₃ | |
| 127 | CH | O | CH₃ | H | C₆H₅ | H | H | CH₃ | |
| 128 | CH | O | CH₃ | CH₃ | CH₃ | H | H | H | |
| 129 | CH | O | CH₃ | CH₃ | CH₃ | H | H | CH₃ | |
| 130 | CH | O | CH₃ | H | CF₃ | H | H | H | |
| 131 | CH | O | CH₃ | H | CH₃ | H | H | E | |
| 132 | CH | O | CH₃ | H | CH₃ | H | E | H | |
| 133 | CH | O | CH₃ | H | CH₃ | H | COCH₃ | H | |
| 134 | CH | O | CH₃ | H | CH₃ | H | H | COCH₃ | |
| 135 | CH | O | CH₃ | H | CH₂Cl | H | H | H | |
| 136 | CH | O | CH₃ | Cl | CH₂Cl | H | H | H | |
| 137 | CH | O | CH₃ | H | CH₂Cl | H | H | CF₃ | |
| 138 | CH | O | CH₃ | H | CH₂Cl | H | H | CH₃ | |
| 139 | CH | O | CH₃ | CH₃ | CH₂OCH₃ | H | H | H | |
| 140 | CH | O | CH₃ | CH₃ | CH₂OCH₃ | H | H | CH₃ | |
| 141 | CH | O | CH₃ | OCH₃ | CH₂Cl | H | H | H | |
| 142 | CH | O | CH₃ | H | CH₂Cl | H | H | E | |
| 143 | CH | O | CH₃ | H | CH₂Cl | H | E | H | |
| 144 | CH | O | CH₃ | H | CH₂Cl | H | COCH₃ | H | |
| 145 | CH | O | CH₃ | H | CH₂Cl | H | H | COCH3 | |
| 146 | CH | O | CH₃ | H | CH₂OCH₂CF₃ | H | H | H | |
| 147 | CH | O | H3 | Cl | CH₂OC₂H₅ | H | H | H | |
| 148 | CH | O | CH₃ | Cl | CH₂OCH₃ | H | H | CH₃ | |
| 149 | CH | O | CH₃ | H | CH₂OCH₃ | H | H | CH₃ | |
| 150 | CH | O | CH₃ | CH₃ | 3-CF₃-C₆H₄ | H | H | H | |
| 151 | CH | O | CH₃ | CH₃ | 3-CH₃-C₆H₄ | H | H | CH₃ | |
| 152 | CH | O | CH₃ | H | 4-CH₃-C₆H₄ | H | H | H | |
| 153 | CH | O | CH₃ | H | 2-Cl-C₆H₄ | H | H | E | |
| 154 | CH | O | CH₃ | H | 3-Cl-C₆H₄ | H | E | H | |
| 155 | CH | O | CH₃ | H | CF₃ | H | COCH₃ | H | |
| 156 | CH | O | CH₃ | Cl | CH₂OCH₃ | H | H | COCH₃ | |
| 157 | CH | O | CH₃ | OCH₃ | CH₂OC₂H₅ | H | H | H | |
| 158 | CH | O | CH₃ | C₂H₅ | CH₂OC₂H₅ | H | CH₃ | H | |
| 159 | CH | O | CH₃ | H | CH₂OC₂H₅ | H | H | CH₃ | |
| 160 | CH | O | CH₃ | Cl | CH₂OC₂H₅ | H | CO₂C₂H₅ | CH₃ | |
| 161 | CH | O | CH₃ | CH₃ | 2-F-C₆H₄ | H | H | H | |
| 162 | CH | O | CH₃ | CH₃ | 3-F-C₆H₄ | H | H | CH₃ | |
| 163 | CH | O | CH₃ | H | 4-F-C₆H₄ | H | H | H | |
| 164 | CH | O | CH₃ | H | CH₂OC₂H₅ | H | H | E | |
| 165 | CH | O | CH₃ | H | CH₂OC₂H₅ | H | E | H | |
| 166 | CH | 0 | CH₃ | H | CH₂OC₂H₅ | H | COCH₃ | H | |
| 167 | CH | O | CH₃ | H | CH₂OC₂H₅ | H | H | COCH₃ | |
| 168 | CH | O | CH₃ | H | CH₂OCH₂CF₃ | H | H | H | |
| 169 | CH | O | CH₃ | Cl | CH₂OCH₂CF3 | H | H | H | |
| 170 | CH | O | CH₃ | H | CF₃ | H | H | CH₃ | |
| 171 | CH | O | CH₃ | H | CH₂OCH₂CF₃ | H | H | CH₃ | |
| 172 | CH | O | CH₃ | CH₃ | CH₂OCH₂CF₃ | H | H | H | |
| 173 | CH | O | CH₃ | CH₃ | -CH₂OPh | H | H | CH₃ | |
| 174 | CH | O | CH₃ | H | -CH₂OPh | H | H | H | |
| 175 | CH | O | CH₃ | H | CH₂OCH₂Ph | H | H | E | |
| 176 | CH | O | CH₃ | H | CH₂OCH₂Ph | H | E | H | |
| 177 | CH | O | CH₃ | H | 4-Cl-C₆H₅ | H | COCH₃ | H | |
| 178 | CH | O | CH₃ | H | 4-Cl-C₆H₅ | H | H | COCH₃ | |
| 179 | CH | O | CH₃ | H | M | H | CO₂C₂H₅ | H | |
| 180 | CH | O | CH₃ | H | M | H | H | H | |
| 181 | CH | O | CH₃ | Cl | M | H | H | CH₃ | |
| 182 | CH | O | CH₃ | H | M | H | H | CH₃ | |
| 183 | CH | O | CH₃ | CH₃ | M | H | H | H | |
| 184 | N | O | CH₃ | H | CH₃ | H | H | H | |
| 185 | N | O | CH₃ | H | C₆H₅ | H | H | Cl | |
| 186 | N | O | CH₃ | CH₃ | CH₃ | H | H | H | |
| 187 | N | O | CH₃ | CH₃ | CH₃ | H | H | H | |
| 188 | N | O | CH₃ | H | CF₃ | H | H | Cl | |
| 189 | N | O | CH₃ | CH₃ | CH₃ | H | H | CH₃ | |
| 190 | N | O | CH₃ | H | CH₃ | H | E | CO₂CH₃ | |
| 191 | N | O | CH₃ | H | CH₃ | H | COCH₃ | CO₂CH₃ | |
| 192 | N | O | CH₃ | H | CH₃ | H | H | CO₂CH₃ | |
| 193 | N | O | CH₃ | H | CH₂Cl | H | H | CO₂CH₃ | |
| 194 | N | O | CH₃ | H | H | H | H | H | |
| 195 | N | O | CH₃ | H | CH₂Cl | H | H | CF₃ | |
| 196 | N | O | CH₃ | H | 3-CF₃-C₆H₄ | H | H | CF₃ | |
| 197 | N | O | CH₃ | CH₃ | 3-CH₃-C₆H₄ | H | H | CF₃ | |
| 198 | N | O | CH₃ | CH₃ | 4-CH₃-C₆H₄ | H | H | CF₃ | |
| 199 | N | O | CH₃ | H | CH₂Cl | H | H | H | |
| 200 | N | O | CH₃ | H | CH₂Cl | H | H | E | |
| 201 | N | O | CH₃ | H | CH₂Cl | H | E | H | |
| 202 | N | O | CH₃ | H | CH₂Cl | H | COCH₃ | H | |
| 203 | N | O | CH₃ | H | CH₂Cl | H | H | COCH3 | |
| 204 | N | O | CH₃ | H | CH₂OCH₃ | H | H | CH₂N(CH₃)₂ | |
| 205 | N | O | CH₃ | Cl | CH₂OCH₃ | H | H | CH₂N(CH₃)₂ | |
| 206 | N | O | CH₃ | CH₃ | CH₂OCH₃ | H | H | CH₂N(CH₃)₂ | |
| 207 | N | O | CH₃ | H | CH₂OCH₃ | H | H | F | |
| 208 | N | O | CH₃ | CH₃ | CH₂OCH₃ | H | H | F | |
| 209 | N | O | CH₃ | CH₃ | CH₂OCH₃ | H | CO₂CH₃ | CH₂N(CH₃)₂ | |
| 210 | N | O | CH₃ | H | CH₂OCH₃ | H | H | H | |
| 211 | N | O | CH₃ | H | CH₂OCH₃ | H | H | E | |
| 212 | N | O | CH₃ | H | 3-CF₃-C₆H₄ | H | E | H | |
| 213 | N | O | CH₃ | H | 3-CH₃-C₆H₄ | H | COCH₃ | H | |
| 214 | N | O | CH₃ | H | 4-CH₃-C₆H₄ | H | H | COCH₃ | |
| 215 | N | O | CH₃ | Cl | CH₂OC₂H₅ | H | H | H | |
| 216 | N | O | CH₃ | H | CH₂OC₂H₅ | H | H | H | |
| 217 | N | O | CH₃ | H | CH₂OC₂H₅ | H | H | CH₃ | |
| 218 | N | O | CH₃ | H | 3-OCH₃-C₆H₄ | H | H | CH₃ | |
| 219 | N | O | CH₃ | CH₃ | 4-OCH₃-C₆H₄ | H | H | H | |
| 220 | N | O | CH₃ | CH₃ | 2-OCH₃-C₆H₄ | H | H | CH₃ | |
| 221 | N | O | CH₃ | H | CH₂OC₂H₅ | H | H | Cl | |
| 222 | N | O | CH₃ | H | CH₂OC₂H₅ | H | H | E | |
| 223 | N | O | CH₃ | H | M | H | E | H | |
| 224 | N | O | CH₃ | H | 3-CF₃-C₆H₄ | H | COCH₃ | H | |
| 225 | N | O | CH₃ | H | 3-CH₃-C₆H₄ | H | H | COCH₃ | |
| 226 | N | O | CH₃ | H | 4-CH₃-C₆H₄ | H | H | H | |
| 227 | N | O | CH₃ | H | 2-Cl-C₆H₄ | H | H | H | |
| 228 | N | O | CH₃ | H | 3-Cl-C₆H₄ | H | H | CH₃ | |
| 229 | N | O | CH₃ | H | CH₂OCH₂CF₃ | H | H | CH₃ | |
| 230 | N | O | CH₃ | CH₃ | CH₂OCH₂CF₃ | H | H | H | |
| 231 | N | O | CH₃ | CH₃ | -CH₂OPh | H | H | CH₃ | |
| 232 | N | O | CH₃ | H | -CH₂OPh | H | H | H | |
| 233 | N | O | CH₃ | H | CH₂OCH₂Ph | H | H | E | |
| 234 | N | O | CH₃ | H | CH₂OCH₂Ph | H | E | H | |
| 235 | N | O | CH₃ | H | 4-Cl-C₆H₄ | H | COCH₃ | H | |
| 236 | CH | O | H | C₃H₇i | CH₃ | H | H | H | oil |
| 237 | CH | O | H | n-C₄H₉ | CH₃ | H | H | H | 117-118 |
| 238 | CH | O | H | n-C₅H₁₁ | CH₃ | H | H | H | |
| 239 | CH | O | H | C₂H₄Pr i | CH₃ | H | H | H | oil |
| 240 | CH | O | H | n-C₆H₁₃ | CH₃ | H | H | H | 113-115 |
| 241 | CH | O | H | H | n-C₄H₉ | H | H | H | |
| 242 | CH | O | H | H | n-C₅H₁₁ | H | H | H | |
| 243 | CH | O | H | H | CH(CH₃)₂ | H | H | CH₃ | 110-112 |
| 244 | CH | O | H | n-C₃H₇ | n-C₃H₇ | H | H | H | 112-114 |
| 245 | N | O | H | Cl | n-C₃H₇ | H | H | H | 136-138 |
| 246 | N | O | H | Cl | C₆H₅ | H | H | H | 166-168 |
| 247 | N | O | H | n-C₃H₇ | CH₃ | H | H | H | 121-122 |
| 248 | N | O | H | n-C₄H₉ | CH₃ | H | H | H | 100-102 |
| 249 | N | O | H | n-C₆H₁₃ | CH₃ | H | H | H | 75-78 |
| 250 | CH | O | H | CH₃ | n-C₄H₉ | H | H | H | |
| 251 | CH | O | H | C₂H₅ | n-C₄H₉ | H | H | H | |
| 252 | CH | O | H | C₃H₇ | n-C₄H₉ | H | H | H | |
| 253 | CH | O | H | i-C₃H₇ | n-C₄H₉ | H | H | H | |
| 254 | CH | O | H | n-C₄H₉ | n-C₄H₉ | H | H | H | |
| 255 | CH | O | H | CH₃ | n-C₅H₁₁ | H | H | H | |
| 256 | CH | O | H | C₂H₅ | n-C₅H₁₁ | H | H | H | |
| 257 | CH | O | H | C₃H₇ | n-C₅H₁₁ | H | H | H | |
| 258 | CH | O | H | i-C₃H₇ | n-C₅H₁₁ | H | H | H | |
| 259 | CH | O | H | n-C₄H₉ | n-C₅H₁₁ | H | H | H | |
| 260 | CH | O | H | H | n-C₆H₁₃ | H | H | H | |
| 261 | CH | O | H | CH₃ | n-C₆H₁₃ | H | H | H | |
| 262 | CH | O | H | C₂H₅ | n-C₆H₁₃ | H | H | H | |
| 263 | CH | O | H | C₃H₇ | n-C₆H₁₃ | H | H | H | |
| 264 | CH | O | H | i-C₃H₇ | n-C₆H₁₃ | H | H | H | |
| 265 | CH | O | H | n-C₄H₉ | n-C₆H₁₃ | H | H | H | |
| 266 | N | O | H | CH₃ | n-C₄H₉ | H | H | H | |
| 267 | N | O | H | C₂H₅ | n-C₄H₉ | H | H | H | |
| 268 | N | O | H | C₃H₇ | n-C₄H₉ | H | H | H | |
| 269 | N | O | H | i-C₃H₇ | n-C₄H₉ | H | H | H | |
| 270 | N | O | H | n-C₄H₉ | n-C₄H₉ | H | H | H | |
| 271 | N | O | H | CH₃ | n-C₅H₁₁ | H | H | H | |
| 272 | N | O | H | C₂H₅ | n-C₅H₁₁ | H | H | H | |
| 273 | N | O | H | C₃H₇ | n-C₅H₁₁ | H | H | H | |
| 274 | N | O | H | i-C₃H₇ | n-C₅H₁₁ | H | H | H | |
| 275 | N | O | H | n-C₄H₉ | n-C₅H₁₁ | H | H | H | |
| 276 | N | O | H | H | n-C₆H₁₃ | H | H | H | |
| 277 | N | O | H | CH₃ | n-C₆H₁₃ | H | H | H | |
| 278 | N | O | H | C₂H₅ | n-C₆H₁₃ | H | H | H | |
| 279 | N | O | H | C₃H₇ | n-C₆H₁₃ | H | H | H | |
| 280 | N | O | H | i-C₃H₇ | n-C₆H₁₃ | H | H | H | |
| 281 | N | O | H | n-C₄H₉ | n-C₆H₁₃ | H | H | H | |
| 282 | CH | O | H | H | CH₂-Ph-4-Cl | H | H | H | |
| 283 | CH | O | H | CH₃ | CH₂-Ph-4-Cl | H | H | H | |
| 284 | CH | O | H | C₂H₅ | CH₂-Ph-4-Cl | H | H | H | |
| 285 | CH | O | H | CH₂-Ph-4-Cl | CH₃ | H | H | H | |
| 286 | CH | O | H | CH₂-Ph-4-Cl | C₂H₅ | H | H | H | |
| 287 | CH | O | H | CH₂-Ph-4-Cl | C₃H₇ | H | H | H | |
| 288 | CH | O | H | CH₃ | CF₃ | H | H | H | |
| 289 | CH | O | H | Cl | CF₃ | H | H | H | |
| 290 | CH | O | H | C₂H₅ | CF₃ | H | H | H | |
| 291 | CH | O | H | n-C₃H₇ | CF₃ | H | H | H | |
| 292 | CH | O | H | n-C₄H₉ | CF₃ | H | H | H | |
| 293 | CH | O | H | H | CH₂CH₂-Ph | H | H | H | |
| 294 | CH | O | H | CH₃ | -4-Cl | H | H | H | |
| 295 | CH | O | H | H | CH₂Bu-t | H | H | H | |
| 296 | CH | O | H | CH₃ | CH₂Bu-t | H | H | H | |
| 297 | CH | O | H | n-C₃H₇ | CH₂Bu-t | H | H | H | |
| 298 | CH | O | H | CH₂Bu-t | CH₃ | H | H | H | |
| 299 | CH | O | H | | CH₃ | H | H | H | |
| 300 | CH | O | H | CH₂CH₂-Ph -4-Cl | C₂H₅ | H | H | H | |
| 301 | CH | O | H | | C₃H₇ | H | H | H | |
| 302 | CH | O | H | CO₂CH₃ | CH₃ | H | H | H | |
| 303 | CH | O | H | CO₂CH₃ | CF₃ | H | H | H | |
| 304 | CH | O | H | CO₂C₂H₅ | C₂H₅ | H | H | H | |
| 305 | CH | O | H | CO₂C₂H₅ | n-C₃H₇ | H | H | H | |
| 306 | CH | O | H | CH₃ | CO₂CH₃ | H | H | H | |

| | | | | | | | | | |
|---|---|---|---|---|---|---|---|---|---|
| * stands for melting point. °C is the unit. | | | | | | | | | |

The present invention also includes preparation of benzopyrone compounds and their isomers having general formula (I).

The compounds of formula I can be easily prepared by reaction of the benzylhalide having general formula (II) with benzopyrone compounds containing hydroxy group having general formula (III) under base condition according to the scheme I. 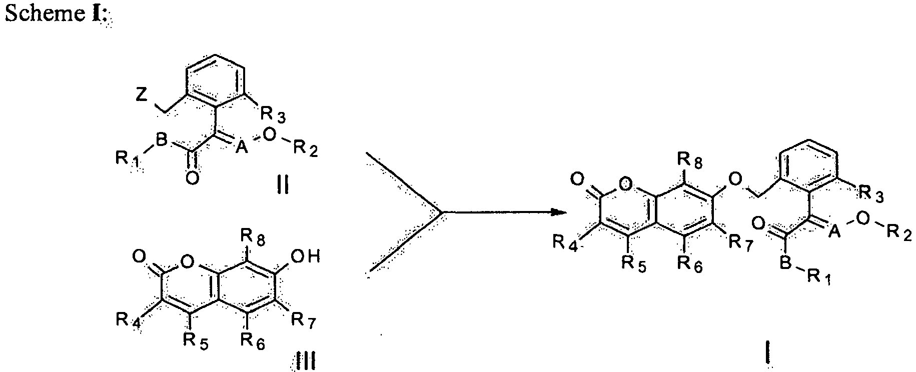
wherein: Z is leaving group, such as halogen (Cl, Br, or I).

R₁, R₂, R₃, R₄, R₅, R₆, R₇, R₈, A, B, is as defined above.

Preparation condition of compounds having general formula **(I):** In proper solvent, hydroxylbenzopyrone compounds having general formula (III) are treated with proper base to become salts, then the compound having general formula (II) is added into the mixture, the reaction is carried out at proper temperature. After reaction is completed, the target compound **I** is obtained by normal way.

The proper solvent mentioned may be selected from the following ones, such as tetrahydrofuran, acetonitrile, toluene, xylene, benzene, DMF, DMSO, acetone or butanone and so on.

The proper base mentioned may be selected from the following ones, such as potassium hydroxide, sodium hydroxide, potassium carbonate, sodium carbonate, sodium bicarbonate, triethylamine, pyridine or sodium hydride and so on.

The proper temperature mentioned is from room temperature to boiling point of solvent. Normal temperature is from 20 to 100°C.

The reaction may be finished in the course of 30 minutes - 20 hours, generally 1-10 hours.

The reaction can be monitored by Thin-Layer Chromatography.

The intermediates of general formula (II) can be prepared according to the known methods , refer to US Pat. No.4723034 and US Pat. No.5554578.

Some of the hydroxylbenzopyrone compounds having general formula (III) are available from the chemical companies, and are also prepared according to the methods reported in Journal of Medicinal Chemistry, 2001, 44(5), 664-671, by the reaction R₅COCHR₄CO₂CH₃(C₂H₅) with substituted resorcinol. Moreover, the compound may be straightly used to prepare the target compounds without further purification. Some of the hydroxylbnezopyrone compounds having general formula (III) synthesized are showed in table **2**.

**Table 2**

| III | **R₄** | **R₅** | **R₆** | **R₇** | **R₈** | **Physical property** * |
|---|---|---|---|---|---|---|
| **III-1** | H | CH₃ | H | H | COCH₃ | 158-160 |
| **III-2** | H | CH₃ | H | H | C(=NOMe)CH₃ | 129-140 |
| **III-4** | H | CH₃ | H | H | CH₃ | 256-258 |
| **III-5** | Cl | CH₃ | H | H | H | 230-234 |
| **III-6** | H | CF₃ | H | H | H | 180-183 |
| **III-7** | C₆H₅CH₂ | CH₃ | H | H | H | 208-212 |
| **III-8** | H | 4-F-C₆H₄ | H | H | H | 256-262 |
| **III-9** | *H* | *3, 4-(MeO)₂C₆H₄* | H | H | *H* | *184-188* |
| **III-10** | F | CH₃ | H | H | H | 203-206 |
| **III-11** | H | C₆H₅ | H | H | H | 240-242 |
| **III-12** | H | C₆H₅ | H | H | CH₃ | 260-262 |
| **III-13** | Cl | C₆H₅ | H | H | H | 188-190 |
| **III-14** | CH₃ | CH₃ | H | H | H | 118-120 |
| **III-15** | CH₃ | CH₃ | H | H | CH₃ | 218-222 |
| **III-16** | H | n-C₃H₇ | H | H | CH₃ | 176-178 |
| **III-17** | Cl | n-C₃H₇ | H | H | H | 148-150 |
| **III-18** | H | i-C₃H₇ | H | H | H | 160-162 |
| **III-19** | n-C₆H₁₃ | CH₃ | H | H | H | 170-172 |
| **III-20** | i-C₃H₇CH₂CH₂ | CH₃ | H | H | H | 101-102 |
| **III-21** | n-C₄H₉ | CH₃ | H | H | H | 134-136 |
| **III-22** | n-C₃H₇ | CH₃ | H | H | H | 142-144 |
| **III-23** | H | CH₂OCH₃ | H | H | H | 186-190 |

| | | | | | | |
|---|---|---|---|---|---|---|
| * stands for melting point. °C is the unit. | | | | | | |

The present invention also provides compositions which are insecticides and fungicides. The active ingredients of the composition are the compounds having general formula (I), wherein the active ones being present in a total amount of 0.1 to 99% by weight, the rest being the acceptable carrier by agriculture.

The present invention, further more, provides preparation method of the said composition thereon. The compounds of general formula (I) and the carrier are mixed. The said composition may be a single component compound or mixture of compounds with several components.

The carrier in the invention accords to the requirements: it is easy to apply to the sites being to be treated for the carrier after it is confected with active component. For example, the sites could be plant, seed or soil convenient for store, transport or operation. The carrier could be solid or liquid, including the liquid which usually turns from gas condition under pressure. And the carriers which are used to confect insecticidal, bactericidal composition are applied.

Suitable solid carriers include natural and synthetic clays and silicates, for example diatomaceous earths, talcs, magnesium aluminium silicates, aluminium silicates(kaolin), montmorillonites and micas; calcium carbonate; calcium sulphate; ammonium sulphate; synthetic silicon oxides and synthetic calcium silicates or aluminium silicates; elements, for example carbon and sulphur; natural and synthetic resins, for example coumarone resins, polyvinyl chloride, and styrene polymers or copolymers; solid polychlorophenols; bitumen; waxes, beeswax or paraffin wax for instance.

Suitable liquid carriers include water, alcohols such as isopropanol or alcohol; ketones such as acetone, methyl ethyl ketone, methyl isopropy ketone or cyclohexanone; ethers; aromatics such as benzene, xylene, toluene; petroleum fractions such as kerosene or mineral oils, chlorinated aliphatic hydrocarbons such as carbon tetrachloride, tetrachloride ethylene or trichloride ethylene. Mixtures of these different liquids generally are often suitable.

The compositions of insecticides and fungicides are often formulated and transported in a concentrated form which is subsequently diluted by the user before application. The presence of small amounts of surpfactant facilitates this process of dilution. Thus preferably at least one carrier in a composition according to the invention is a surfactant. For example the composition may contain at least two carriers, at least one of which is a surfactant.

A surfactant may be an emulsifier, a dispersant or a wetting agent; it may be nonionic or ionic. Examples of suitable surfactant include the sodium or calcium salts of polyacrylic acids and lignin sulphonic acids; the condensation products of fatty acids or aliphatic amines or amides containing at least 12 carbon atoms in the molecule with ethylene oxide and/or propylene oxide; fatty acid esters of glycol, sorbic alcohol, sucrose or pentaerythritol and condensations of these with ethylene oxide and/or propylene oxide; condensation products of fatty alcohol or alkyl phenols such as p-octylphenol or p-octylcresol, with ethylene-oxide and/or propylene oxide; sulphates or sulphonates of these condensation products; alkaline metal salts or alkaline earth metal salts, preferably sodium salts, of sulphuric or sulphonic acid esters containing at least 10 carbon atoms in the molecule, for example sodium lauryl sulphate, sodium secondary alkyl sulphates, sodium salts of sulphonated castor oil, and sodium alkylaryl sulphonates such as sodium dodecylbenzene sulphonate.

Examples of compositions and formulations according to the invention are wettable powder, Dustable powder, granule and aqueous solution; emulsifiable concentrate, emulsion, suspension concentrate, aerosol composition and fumigant. Wettable powder usually contains 25, 50 or 75% weight(ab.w) of active ingredient and usually contain in addition to solid inert carrier, 3-10% w of a dispersant and, where necessary, 0-10% w of stabiliser(s) and/or other additives such as penetrants or stickers. Dustable powder are usually formulated as a dust concentrate having a similar composition to that of a wettable powder but a dispersant, and are diluted with further solid carrier to give a composition usually containing 0.5-10% weight of active ingredient. Granules are usually prepared to have a size between 10 and 100 BS mesh (1.676-0.152 mm), and may be manufactured by agglomeration or impregnation techniques. Generally, granules contain 0.5-75% w active ingredient and 0-10% weight of additives such as stabilisers, surfactants, slow release modifiers. The so-called "dry flowable powders" consist of relatively small granules having a relatively high concentration of active ingredient. Emulsifiable concentrates usually contain, in addition to a solvent and, when necessary, co-solvent, 1-50% weight /volume(ab. w/v) active ingredient, 2-20% w/v emulsifiers and 0-20% w/v of other additives such as stabilisers, penetrants and corrosion inhibitors. Suspension concentrates are usually contain 10-75% w active ingredient, 0.5-15% w of dispersing agents,0.1-10% w of other additives such as defoamers, corrosion inhibitors, stabilisers, penetrants and stickers.

Aqueous dispersant and emulsions, for example compositions obtained by diluting a wettable powder or a concentrate according to the invention with water, also lie within the scope of the invention. The said emulsions may be of the water-in-oil or of the oil-in-water type.

The composition to which one or more other fungicides are added has wider spectrum activity than single compound having general formula (**I**). In addition, other fungicides may have synergistic effect on the fungicidal activity of the compound having general formula(I). The compound having general formula (I) can also be used with other insecticides, or with another fungicide and other insecticides simultaneously.

This invention has the following advantages:

The compounds of present invention have very good insecticide activity, and may be used to control insects such as armyworm, diamond backmoth, aphids, carmine spider mite, two-spotted spider mite, lady beetles, mites and culex mosquitoes, especially for lady beetles and culex mosquitoes. All these attributes are suitable for integrated insect management.

The compounds of present invention have wide spectrum fungicidal activity, and may be used to control diseases in all sorts of plants caused by by oomycete, basidiomycete, ascomycete pathogens, and it may also provide good control efficacy at very low rate because of the high activity. These compounds have penetration activity and can be used as soil and foliar fungicides. They can provide satisfied control of grape downy mildew, rice sheath blight, rice blast, tomato early blight, tomato late blight, wheat leaf rust, wheat leaf blotch, wheat powdery mildew, cucumber powdery mildew, cucumber downy mildew and cucumber grey mold

### DESCRIPTION OF THE INVENTION IN DETAIL

The following examples are illustrative of the present invention.

### Preparation Example

### Example 1 the preparation of compound 1

A reaction flask was charged a suspension of 60% sodium hydride 0.84 g (washed with petroleum ether), and then 30 ml of dry *N,N*-dimethylformamide (DMF) was added, the mixture was stirred at room temperature for 30 minutes. To this agitated suspension, 1.7 g of 7-hydroxycoumarin was added, the mixture was agitated continuously till to no gas emerging. 3.0g of methyl (*E*)-α-[2-(bromomethyl)phenyl]-β-methoxyacrylate was added to the reaction mixture and they were agitated continuously for 3 hours at room temperature. The reaction mixture was poured into ice water, extracted with ethyl acetate 3 times. The combined extracts were washed with brine 3 times, dried, filtered and concentrated under vacuum, to obtain the crude oil product 5g. This was subjected to column chromatography to obtain 2.8 g of compound 1 as a faint red-yellow oily substance in 76.5% yield.
¹HNMR(300MHz, internal standard=TMS, CDCl₃): δppm 3.69(3H, s), 3.88(3H, s), 5.04(2H, s), 6.19-6.23(1H, d), 6.77(1H, s), 6.83-6.87(1H, d), 7.18-7.20(1H, m), 7.26-7.34(4H, m), 7.48-7.64(2H, m).

### Example 2 the preparation of compound 2

A reaction flask was charged a suspension of 60% sodium hydride 0.45 g (washed with petroleum ether) ,and then 20 ml of dry *N,N*-dimethylformamide (DMF) was added ,the mixture was stirred at room temperature for 30 minutes. To this agitated suspension, 1.0 g of 7-hydroxy-4-methylcoumarin was added, the mixture was agitated continuously till to no gas emerging. 1.66g of methyl (*E*)-α-[2-(chloromethyl)phenyl]-β-methoxyacrylate was added to the reaction mixture and they were agitated continuously for 3 hours at room temperature.The reaction mixture was poured into ice water, extracted with ethyl acetate 3 times. The combined extracts were washed with brine 3 times. dried, filtered and concentrated, to obtain the crude product, as a yellow solid substance. This was subjected to column chromatography, using a 1:2 mixture of ethyl acetate and petroleum ether as the eluting solution to obtain 1.73 g of compound 2, with melting point of 140-143°C in 80% yield.
¹HNMR(300MHz, internal standard=TMS, CDCl₃): δppm 2.38(3H, s), 3.74(3H, s), 3.89(3H, s), 5.04(2H, s), 6.11 (1H, s), 6.77(1H, s), 6.85-6.89(1H, d), 7.17-7.20(1H, m), 7.32-7.35(2H, m), 7.49-7.52(2H, m), 7.64(1H, s).

### Example 3 the preparation of compound 72

A reaction flask was added 1.2g of K₂CO₃, 1.0g of 7-hydroxy-4-methylcoumarin, 1.70g of methyl (E)-2-(bromomethyl)-α-(methoxyimino)benzeneacetate and 20ml Butanone, the reaction mixture was refluxed and agitated continuously for 5 hours. The reaction mixture was poured into ice water, extracted with ethyl acetate 3 times. The combined extracts were washed with brine 3 times, dried, filtered and concentrated under vacuum, to obtain the crude product, as a yellow solid substance. This was subjected to column chromatography, using a 1:2 mixture of ethyl acetate and petroleum ether as the eluting solution to obtain1.77 g of compound 72, with melting point of 150-152°C in 83% yield.
¹HNMR(300MHz, internal standard=TMS, CDCl₃): δppm 2.39(3H, s), 3.87(3H, s), 4.05(3H, s), 5.02(2H, s), 6.13(1H, s), 6. 80-6.86(2H, m), 7.23-7.26(1H, m), 7.43-7.49(4H, m).

The following compounds were prepared analogously. ¹HNMR of other compounds are provided as follows (300MHz, internal standard=TMS, CDCl₃):
Compound 3: δppm 2.36(3H, s), 2.37(3H, s), 3.72(3H, s), 3.84(3H, s), 5.09(2H, s), 6.13 (1H, s), 6.75-6.78(1H, d), 7.18-7.21(1H, m), 7.34-7.36(3H, m), 7.50-7.52(1H, m)7.61(1H, s).
Compound 4: δppm 2.41(3H, s), 3.69(3H, s), 3.81(3H, s), 5.08(2H, s), 6.20(1H, s), 6.68-6.71(1H, d), 7.18-7.21(4H, m), 7.32-7.50(5H, m), 7.59(1H, s), 7.92(1H, m).
Compound 5: δppm 2.17(3H, s), 2.35(3H, s), 3.73(3H, s), 3.88(3H, s), 5.02(2H, s), 6.78(1H, s), 6.83-6.85(1H, d), 7.31-7.34(3H, m), 7.45-7.47(2H, d), 7.62(1H, s).
Compound 6: δppm 2.32(3H, s), 2.31-2.36(6H, d), 3.69(3H, s), 3.84(3H, s), 5.07(2H, s), 6.74-6.77(1H, d), 7.17-7.20(1H, m), 7.31-7.36(3H, m), 7.51-7.54(1H, m), 7.61(1H, s).
Compound 12: δppm 2.53(3H, s), 3.74(3H, s), 3.89(3H, s), 5.04(2H, s), 6.78(1H, s), 6.83-6.85(1H, d), 7.18-7.21(1H, m), 7.32-7.35(2H, m), 7.47-7.50(2H, d), 7.64(1H, s).
Compound 17: δppm 1.25-1.32(3H, m), 2.36(3H, s), 2.74-2.76(2H, m), 3.71(3H, s), 3.84(3H, s), 5.08(2H, s), 6.15(1H, s), 6.75-6.78(1H, d), 7.18-7.21(1H, m), 7.33-7.38(3H, m), 7.50-7.54(1H, m), 7.61(1H, s).
Compound 18: δppm 1.10-1.15(3H, t), 2.37(3H, s), 2.60-2.68(2H, q), 3.74(3H, s), 3.89(3H, s), 5.03(2H, s), 6.76 (1H, d), 6.84-6.88(1H, dd), 7.18-7.21(1H, m), 7.32-7.35(2H, m), 7.45-7.53(2H, m), 7.63(1H, s).
Compound 19: δppm 3.48(3H, s), 3.74(3H, s), 3.89(3H, s), 4.56(2H, s), 5.04(2H, s), 6.34(1H, s), 6.79(1H, d), 6.84-6.88(1H, dd), 7.18-7.21(1H, m), 7.30-7.36(2H, m), 7.41-7.44(1H, d), 7.48-7.51 (1H, m), 7.64(1H, s).
Compound 24: δppm 3.72(3H, s), 3.92(3H, s), 5.10(2H, s), 6.78(1H, s), 6.94-7.21(1H, d), 7.22(1H, m), 7.33-7.35(2H, m), 7.36-7.45(2H, m), 7.66(1H, s), 8.13(1H, s).
Compound 25: δppm 2.36(3H, d), 2.62(3H, d), 3.71(3H, s), 3.84(3H, s), 5.09(2H, s), 6.82(1H, d), 7.19-7.21(1H, m), 7.33-7.35(3H, m), 7.36-7.37(1H, m), 7.61(1H, s).
Compound 26: δppm 1.25-1.30(6H, m), 3.20-3.23(1H, m), 3.74(3H, s), 3.91(3H, s), 5.04(2H, s), 6.15(1H, s), 6.790-6.799(1H, d), 6.80-6.90(1H, m), 7.18-7.23(1H, m), 7.32-7.37(2H, m), 7.48-7.57(2H, m), 7.64(1H, s).
Compound 27: δppm 0.95-1.00(3H, t), 1.58(2H, m), 2.36(3H, s), 2.58(2H, t), 3.73(3H, s), 3.89(3H, s), 5.02(2H, s), 6.75(1H, d), 6.84-6.88(1H, dd), 7.18(1H, m), 7.31-7.34(1H, m), 7.47-7.51(2H, m), 7.63(1H, s).
Compound 29: δppm 3.74(3H, s), 3.90(3H, s), 5.06(2H, s,), 6.17(1H, s), 6.80-6.85 (2H, m), 7.24-7.26(1H, m), 7.28-7.35(5H, m), 7.38-7.51(3H, m), 7.66(1H, s).
Compound 32: δppm 3.73(3H, s), 3.90(3H, s), 5.05(2H, s), 6.75-6.78 (1H, dd), 6.84-6.85(1H, d), 6.94-6.98(1H, d), 7.19-7.21(1H, m), 7.30-7.35(4H, m), 7.53-7.55(4H, m), 7.65(1H, s).
Compound 33: δppm 1.27-1.32(3H, m), 2.74-2.77(2H, m), 3.74(3H, s), 3.89(3H, s), 5.04(2H, s), 6.13(1H, s), 6.78-6.79(1H, d), 6.85-6.89(1H, m), 7.18-7.21(1H, m), 7.32-7.35(2H, m), 7.48-7.52(2H, m), 7.64(1H, s).
Compound 34: δppm 0.90-1.03(3H, m), 1.67-1.72(2H, m), 2.65-2.70(2H, m), 3.73(3H, s), 3.89(3H, s), 5.04(2H, s), 6.10(1H, s), 6.78-6.79(1H, d), 6.85-6.89(1H, m), 7.19-7.21(1H, m), 7.33-7.35(2H, m), 7.47-7.51(2H, m), 7.64(1H, s).
Compound 35: δppm 1.00-1.25(3H, m), 1.69-1.72(2H, m), 2.36(3H, s), 2.65-2.70(2H, m), 3.71(3H, s), 3.84(3H, s), 5.08(2H, s), 6.12(1H, s), 6.75-6.78(1H, d), 7.21-7.26(1H, m), 7.33-7.38(3H, m), 7.50-7.53(1H, m), 7.61(1H, s).
Compound 36: δppm 0.97(3H, t), 1.66(2H, m), 2.67(3H, s), 3.74(3H, s), 3.89(3H, s), 5.04(2H, s), 6.78(1H, d), 6.85-6.88(1H, dd), 7.22(1H, m), 7.33-7.35(2H, m), 7.46-7.49(2H, m), 7.64(1H, s).
Compound 37: δppm 1.05(3H, m), 1.57-1.64(2H, m), 2.16(3H, s), 2.71-2.76(2H, t), 3.70(3H, s), 3.83(3H, s), 5.02(2H, s), 6.78(1H, d), 6.87 (1H, m), 7.20(1H, m), 7.32(2H, m), 7.45(2H, m), 7.64(1H, s).
Compound 38: δppm(DMSO-*d₆*) 3.65(3H, s), 3.88(3H, s), 5.03(2H, s), 6.15(1H, s), 6.83-6.87(1H, dd), 6.91(1H, d), 7.09-7.17(2H, m), 7.23-7.35(4H, m), 7.43-7.46(1H, m), 7.51-7.55(2H, m), 7.66(1H, s).
Compound 41: δppm 3.74(3H, s), 3.91(3H, s), 5.06(2H, s), 6.20(1H, s), 6.86(2H, m), 7.22(2H, m), 7.33- 7.36 (2H, m), 7.56(3H, m), 7.66(1H, s), 7.77(2H, d).
Compound 50: δppm 2.34(3H, s), 3.74(3H, s), 3.89(3H, s), 5.04(2H, s), 6.78-6.79(1H, d), 6.92-6.96(1H, dd), 7.18-7.21(1H, m), 7.32-7.35(2H, m), 7.41-7.44(1H, d), 7.48-7.51(1H, m), 7.65(1H, s).
Compound 52: δppm 3.74(3H, s), 3.90(3H, s), 5.06(2H, s), 6.20(1H, s), 6.80-6.86(1H, m), 7.18-7.22(1H, m), 7.32-7.37(4H, m), 7.41-7.44(2H, m), 7.50-7.52(4H, m), 7.65(1H, s).
Compound 58: δppm 3.74(3H, s), 3.91(6H, d), 3.96(3H, s), 5.06(2H, s), 6.19 (1H, s), 6.81-6.82(1H, m), 6.85 (1H, s), 6.93-7.04(3H, m), 7.19-7.22(1H, m), 7.33-7.36(2H, m), 7.44-7.52(2H, m), 7.66(1H, s).
Compound 68: δppm(DMSO-*d₆*) 2.49(3H, s), 3.66(3H, s), 3.89(3H, s), 3.92(2H, s), 5.00(2H, s), 6.78-6.79(1H, d), 6.85-6.89(1H, dd), 7.10-7.22(6H, m), 7.26-7.29(2H, m), 7.42(1H, m), 7.61-7.66(2H, m).
Compound 71: δppm 2.54(3H, s), 3.87(3H, s), 4.04(3H, s), 5.02(2H, s), 6.81-6.85(1H, s), 7.26(1H, d), 7.43-7.52(5H, m).
Compound 73: δppm 2.32(3H, s), 2.37(3H, s), 3.84(3H, s), 4.03(3H, s), 5.05(2H, s), 6.13(1H, s), 6.76-6.79(1H, d), 7.26(1H, d), 7.34-7.43(3H, m), 7.45-7.46(1H, d).
Compound 74: δppm 2.18(3H, s), 2.37(3H, s), 3.91(3H, s), 3.98(3H, s), 5.35(2H, s), 6.85(1H, s), 6. 86-6.88(1H, d), 7.26-7.40(3H, m), 7.49-7.52(1H, d), 7.62-7.65(1H, d).
Compound 75: δppm 2.17(3H, s), 2.35(3H, s), 3.86(3H, s), 4.04(3H, s), 5.00(2H, s), 6.78-6.85(2H, m), 7.20-7.25(1H, d), 7.40-7.61 (4H, m).
Compound 80: δppm 2.91-2.93(3H, d), 3.97(3H, s), 5.02(2H, s), 6.23-6.26(1H, d), 6.82-6.86(3H, m), 7.20-7.23(1H, m), 7.34-7.37(1H, d), 7.39-7.45(2H, m), 7.50-7.53(1H, m), 7.61-7.64(1H, d).
Compound 82: δppm 3.87(3H, s), 4.05(3H, s), 5.02(2H, s), 6.23-6.26(1H, d), 6.79-6.85(2H, m), 7.21(1H, d), 7.34-7.37(1H, d), 7.41-7.45(2H, m), 7.47-7.53(1H, m), 7.61-7.64(1H, d).
Compound 236: δppm 1.32-1.36(6H, m), 2.39(3H, s), 3.27(1H, m), 3.74(3H, s), 3.89(3H, s), 5.03(2H, s), 6.72-6.73(1H, d), 6.83-6.87(1H, dd), 7.17-7.20(1H, m), 7.31-7.34(2H, m), 7.46-7.52(2H, m), 7.63(1H, s).
Compound 237: δppm 0.93(3H, m), 1.45(4H, m), 2.35(3H, s), 2.60(2H, t), 3.74(3H, s), 3.89(3H, s), 5.04(2H, s), 6.78(1H, d), 6.84-6.85 (1H, m), 7.18-7.20(1H, m), 7.30-7.35(2H, m), 7.45-7.50(2H, d), 7.64(1H, s).
Compound 239: δppm1.25(6H, m), 1.39(2H, m), 1.63(1H, m), 2.39(3H, s), 2.62(2H, t), 3.72(3H, s), 3.86(3H, s), 5.01(2H, s), 6.78(1H, d), 6.84 (1H, m), 7.20(1H, m), 7.32(2H, m), 7.45(2H, d), 7.64(1H, s).
Compound 240: δppm 0.88(3H, t), 1.42-1.52(8H, m), 2.38(3H, s), 2.64(2H, t), 3.72(3H, s), 3.86(3H, s), 5.01(2H, s), 6.78(1H, d), 6.84 (1H, m), 7.20(1H, m), 7.32(2H, m), 7.45(2H, d), 7.64(1 H, s).
Compound 243: δppm 2.37(3H, s), 3.2-3.6(1H, m), 3.72(3H, s), 3.85(3H, s), 5.09(2H, s), 6.18(1H, s), 6.76-6.79(1H, d), 7.18-7.21(1H, m), 7.34-7.43(3H, m), 7.51-7.54(1H, m), 7.68(1H, 5).
Compound 244: δppm 0.96-1.03(6H, m), 1.58-1.63(4H, m), 2.71-2.79(4H, m), 3.72(3H, s), 3.85(3H, s), 5.00(2H, s), 6.79(1H, d), 6.87 (1H, m), 7.19(1H, m), 7.32(2H, m), 7.45(2H, m), 7.64(1H, s).
Compound 245: δppm 0.86-0.88(3H, m), 1.68-1.75(2H, m), 2.66-2.71(2H, m), 3.87(3H, s), 4.05(3H, s), 5.02(2H, s), 6.80-6.92(3H, m), 7.21-7.26(1H, d), 7.39-7.69(3H, m).
Compound 246: δppm 3.87(3H, s), 4.05(3H, s), 5.02(2H, s), 6.73-6.77(1H, m), 6.87-6.88(1H, d), 6.97-7.00(1H, d), 7.21-7.24(1H, m), 7.28-7.32(2H, m), 7.42-7.57(6H, m).
Compound 247: δppm 0.94(3H, t), 1.46(2H, m), 2.35(3H, s), 2.60(2H, t), 3.74(3H, s), 3.89(3H, s), 5.04(2H, s), 6.78(1H, d), 6.84 (1H, m), 7.20(1H, m), 7.32(2H, m), 7.42-7.45(2H, d), 7.64(1H, s).
Compound 248: δppm 0.94(3H, m), 1.45(4H, m), 2.36(3H, s), 2.60(2H, t), 3.86(3H, s), 4.05(3H, s), 5.00(2H, s), 6.78(1H, d), 6.84 (1H, m), 7.20(1H, m), 7.38-7.45(4H, m).
Compound 249: δppm 0.88(3H, m), 1.48-1.65(8H, m), 2.36(3H, s), 2.62(2H, t), 3.86(3H, s), 4.05(3H, s), 5.00(2H, s), 6.85(1H, m), 6.84 (1H, m), 7.20(1H, m), 7.39-7.45(4H, m).

### Formulation example(weight/weight %)

### Example 5 60% wettable powder

| | |
|---|---|
| Compound 6 | 60% |
| Sodium dodecylnaphthalenesulfate | 2 % |
| Sodium lignosulphonate | 9 % |
| Kaolin | complement to 100% |

All the solid components are well mixed and shattered until the particle size of the active ingredient reaches the standard in order to obtain 60% wettable powder.

### Example 6 35 % emulsion concentrate

| | |
|---|---|
| Compound 1 | 35% |
| Phosphorous acid | 10 % |
| Ethylenoxy aliphatic acid glycerin ester | 15 % |
| Cyclohexanone | complement to 100% |

Phosphorous acid is dissolved in cyclohexanone, then the compound 1 and ethylenoxy aliphatic acid glycerin ester are added, a emulsifiable concentrate in transparent solution is obtained finally.

### Example 7 30 % aqueous Suspension Concentrate

| | |
|---|---|
| Compound 25 | 30% |
| Sodium dodecylnaphthalenesulfate | 4 % |
| Hemicellulose | 2 % |
| Epoxypropane | 8 % |
| Water | complement to 100% |

The mixture of compound 25, 80% of the amount of water should being added and sodium dodecylnaphthalenesulfate are shattered in a mill (1mm ball). Other components are dissolved in the rest water, and are added under stirring.

### Example 8 25 % suspension emulsifier

| | |
|---|---|
| Compound 12 | 25 % |
| Alkylsulphonates (emulsifier 1) | 4% |
| Ethylenoxy aliphatic acid glycerin ester (emulsifier 2) | 2% |
| Calcium dodecylbenzenesulfate (emulsifier 3) | 1.5% |
| Polyethylenoxyalkyl propyl ether (dispersant) | 2.5% |
| Cyclohexanone (solvent 1) | 30% |

Petroleum fractions (boiling point >200°C) (solvent 2) complement to 100%

Compound 12 is dissolved in 80% of the amount of solvent should being added, and then emulsifiers and dispersant are added, the mixture is stirred completely and shattered in a mill(lmm ball). Other solvents are added.

### Test of Biological Activity

### Example 9 Fungicidal activity determination

Determination of fungicidal activities against plant diseases of selected compounds were carried out by following procedure:

Plants were prepared in pot. Technical samples were dissolved in DMF and diluted to required concentration by water. Test solution was sprayed onto potted plant. Pathogen inoculation was carried out after 24 hours then plants were held in growth chambers at constant temperature and moisture for effect. When untreated plant was manifesting a suitable level of disease (after 1 week approximately), assessment was carried out by visual observation.

Part of test results:
At 200 ppm, compound 1, 2, 4, 5, 6,12,18,19, 25, 26, 33, 34, 35, 37, 50, 52, 58, 69, 80, 237, 240, 244, 245, 248 and 249 showed 100% control against cucumber downy mildew, while compounds 3, 24, 36, 38 and 246 showed >95% control.
At 200 ppm, compound 2, 6, 18, 50, 58, 71 and 237 showed 100% control against cucumber grey mold, while 6, 72, 73, 74, 77 and 247 showed >75% control.
At 200 ppm, compound 6, 7, 10 showed 100% against grape downy mildew, while 8 and 77 showed >85% control.
At 200 ppm, compound 3 and 72 showed >85% control against rice sheath blight.
At 200 ppm, compound 6, 8, 10 showed >85% control against rice blast.
At 200 ppm, compound 237, 247 and 248 showed 100% control against wheat powdery mildew, while 9, 72, 82 and 245 showed >70% control.
At 200 ppm, compound 6 showed 100% control against wheat leaf rust, while 7, 10 showed >95% control and 8 showed >75% control.
At 200 ppm, compound 6 >90% and compound 7, 8, 9, 10 and 11 showed >80% control against wheat leaf blotch.
At 200 ppm, compound 6, 7 showed >100% control against tomato early stage blight; 8 and 10 showed >90% control, while11 showed >75% control.
At 200 ppm, compound 6 showed >95% control against tomato late blight, while10 showed >75% control.
At 200ppm, compound 5, 6 showed >95% control against corn leaf blight.

Comparing with the compound JP51 in JP04-182461, test results of some compounds activity against cucumber downy mildew are shown in table 3.

**Table 3 Comparision of fungicidal activity against cucumber downy mildew (50 ppm)**

| Compound | 1 | 2 | 5 | 6 | 12 | 26 | 37 | 52 | 237 | 240 | 244 | 249 | **JP51** |
|---|---|---|---|---|---|---|---|---|---|---|---|---|---|
| control(%) | 100 | 100 | 100 | 100 | 100 | 100 | 100 | 100 | 100 | 100 | 100 | 100 | **20** |

### Example 10

### Determination of insecticidal /acaricidal activity

Numerous insect larvae were put into containers then were fed with treated corn leaves. Potter's spraying tower was used as the sprayer and spraying volume was 1 mL. The spraying pressure was 13.5 lb/in² (9.3 x 10⁴Nm⁻²).

Test result:
At 10 ppm, compound 2, 5, 6 showed 100% control of culex mosquitoes.
At 600 ppm, compound 5 and 6 showed >50% control of army worm, diamond backmoth and green peach aphid.
At 300 ppm, compound 7, 9, 10 showed 100% control of Mexican lady beetle, while compound 7 showed >50% control of two-spotted spider mite.

## Claims

1. A benzopyrone compound of general formula (I): 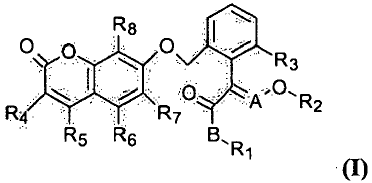 or a stereoisomer thereof, wherein:
A is CH or N;
B is O or S;
R₁ andR₂ are respectively selected from H, C₁-C₁₂ alkyl or C₁-C₁₂ haloalkyl;
R₃ is selected from H, C₁-C₂ alkyl, C₁-C₂ haloalkyl orC₁-C₂ alkoxy;
R₄, R ₅, R ₆, R ₇, and R ₈ may be the same or different, and are selected from: H; halo; CN; NO_{2;} C₁-C₂ alkyl; C₁-C₂ alkenyl; C₁-C₂ alkynyl; C₁-C₂ haloalkyl; C₁-C₂ alkoxy; C₁-C₂ alkylthio; C₁-C₂ alkylsulfonyl; C₁-C₂ alkylcarbonyl; C₁-C₂ alkoxyC₁-C₂alkyl; C₁-C₂ alkoxycarbonyl; C₁-C₂ alkoxycarbonyl C₁-C₂ alkyl; C₁-C₁₂ haloalkoxyC₁-C₁₂ alkyl; amino C₁-C₁₂alkyl in which amino is optionally substituted with up to 2 C₁-C₁₂ alkyl; optionally substituted aryl, aryloxyl, arylC₁-C₁₂ alkyl, arylC₁-C₁₂ alkoxy, aryloxyC₁-C₁₂ alkyl, arylC₁-C₁₂ alkoxylC₁-C₁₂ alkyl, heteroaryl, heteroarylC₁-C₁₂ alkyl, or heteroarylC₁-C₁₂ alkoxyl groups, said optional substituents being up to 3 groups selected from (a) halo, (b) NO₂, (c) C₁-C₆ alkyl, (d) C₁-C₆ haloalkyl, (e) C₁-C₆ alkoxy and (f) C₁-C₆ alkoxyC₁-C₆ alkyl; and groups of the formula: 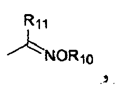
wherein:R₁₀ and R₁₁ are selected from (a) H, (b) C₁-C₁₂ alkyl, (c) aryl and (d) aryl C₁-C₁₂ alkyl.

2. The benzopyrone compound according to the claim 1, wherein:
R₁ and R₂ are respectively selected from H, C₁-C₆ alkyl or C₁-C₆ haloalkyl;
R₃ is selected from H, C₁-C₆ alkyl, C₁-C₆ haloalkyl or C₁-C₆ alkoxy;
R₄, R ₅, R ₆, R ₇, and R ₈ may be the same or different, and are selected from H; halo; CN; NO₂, alkyl; C₂-C₆ alkenyl; C₂-C₆ alkynyl; C₁-C₆ haloalkyl; C₁-C₆ alkoxy; C₁-C₆ alkylthio; C₁-C₆ alkylsulfonyl; C₁-C₆ alkylcarbonyl; C₁-C₆ alkoxyC₁-C₆ alkyl; C₁-C₆ alkoxycarbonyl; C₁-C₆ alkoxycarbonylC₁-C₆ alkyl; C₁-C₆ haloalkoxyC₁-C₆ alkyl; amino C₁-C₆alkyl in which amino is substituted with 0-2 C₁-C₁₂ alkyl; optionally substituted aryl, aryloxyl, arylC₁-C₆ alkyl, arylC₁-C₆ alkoxy, aryloxyC₁-C₆ alkyl, arylC₁-C₆ alkoxylC₁-C₆ alkyl, heteroaryl, heteroarylC₁-C₆ alkyl, or heteroarylC₁-C₆ alkoxyl groups, said optional substituents being up to 3 groups selected from (a) halo, (b) NO₂, (c) C₁-C₂ alkyl, (d) C₁-C₂ haloalkyl, (e) C₁-C₂ alkoxy and (f) C₁-C₂ alkoxyC₁-C₂ alkyl; and groups of the formula: 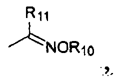 wherein:R₁₀ and R₁₁ are respectively selected from (a) H, (b) C₁-C₆ alkyl, (c) aryl and (d) arylC₁-C₆ alkyl.

3. The benzopyrone compound according to the claim 2, wherein:
B is O;
R₁ and R₂ are both methyl;
R₃ is H or methyl;
Ra, R ₅, R ₆, R ₇, and R ₈ may be the same or different, and are selected from H, halo, CN, NO₂, C₁-C₆ alkyl, C₂-C₆ alkenyl, C₁-C₆ haloalkyl, C₁-C₆ alkoxy, C₁-C₆ alkylcarbonyl, C₁-C₆ alkoxyC₁-C₆ alkyl, C₁-C₆alkoxycarbonyl, C₁-C₆ alkoxycarbonylC₁-C₃alkyl, C₁-C₃ haloalkoxyC₁-C₃ alkyl, amino C₁-C₃alkyl in which amino is optionally substituted with up to 2 C₁-C₃ alkyl; optionally substituted phenyl, phenoxy, phenyl C₁-C₂ alkyl, phenylC₁-C₂ alkoxy, phenoxy C₁-C₂ alkyl, phenylmethyl, phenylmethoxyl, or phenylmethoxy C₁-C₂ alkyl groups, said substitutents being up to 2 groups selected from: (a) halo, (b) NO₂, (c) C₁-C₂ alkyl, (d) C₁-C₂ haloalkyl, (e) C₁-C₂ alkoxy and (f) C₁-C₂ alkoxyC₁-C₂ alkyl; and groups of the formula: 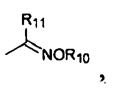 wherein:R₁₀ and R₁₁ are respectively selected from H and C₁-C₆ alkyl.

4. The benzopyrone compound according to the claim 3, wherein:
R₄, R ₅, R ₆, R ₇, and R ₈ may be the same or different, respectively selected from H; Cl; Br; F; CN; C₁-C₆ alkyl; C₁-C₆ haloalkyl; C₁-C₆ alkylcarbonyl; C₁-C₆ alkoxy; C₁-C₆ alkoxyC₁-C₃ alkyl; C₁-C₃ haloalkoxyC₁-C₃ alkyl; amino C₁-C₃alkyl in which amino is optionally substituted with up to 2 C₁-C₃ alkyl; optionally substituted phenyl, phenoxy, phenylmethyl, or phenylmethoxyl, groups, said substitutents being up to 2 groups selected from: (a) halo; (b) NO₂; (c) C₁-C₂ alkyl; (d) C₁-C₂ haloalkyl; (e) C₁-C₂ alkoxy and (f) C₁-C₂ alkoxyC₁-C₂ alkyl; and the groups of formula:

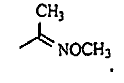

5. A preparation method of a benzopyrone compound according to any preceding claim, wherein the compound of general formula (I) is prepared by reaction of Benzylhalide having general formula (II) with a 7-OH-benzopyrone compound having general formula (III) in the presence of base: 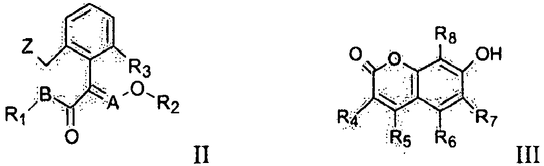 wherein:
Z is a leaving group selected from Cl or Br; and A, B and R₁ to R₈ are as defined in a preceding claim.

6. Use of a benzopyrone compound according to any of claims 1-4 for controlling insects in plants.

7. Use of a benzopyrone compound according to any of claims 1-4 for controlling fungi in plants.

8. A fungicidal and insectidal composition which comprises the compound of claim 1 as an active ingredient, wherein the weight percentage of the active ingredient in the composition is from 0.1 % to 99%.

## Patentansprüche

1. Benzopyron-Verbindung der allgemeinen Formel (I): 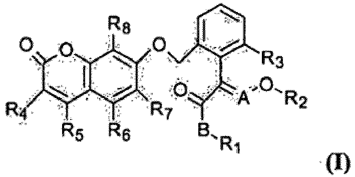 oder ein Stereoisomer davon, worin:
A CH oder N ist;
B O oder S ist;
R₁ und R₂ jeweils unabhängig voneinander aus H, C₁-C₁₂-Alkyl und C₁-C₁₂-Halogenalkyl ausgewählt sind;
R₃ aus H, C₁-C₁₂-Alkyl, C₁-C₁₂-Halogenalkyl oder C₁-C₁₂-Alkoxy ausgewählt ist;
R₄, R₅, R₆, R₇ und R₈ gleich oder unterschiedlich sein können und aus H; Halogen; CN; NO₂; C₁-C₁₂-Alkyl; C₂-C₁₂-Alkenyl; C₂-C₁₂-Alkinyl, C₁-C₁₂-Halogenalkyl, C₁-C₁₂-Alkoxy, C₁-C₁₂-Alkylthio; C₁-C₁₂-Alkylsulfonyl, C₁-C₁₂-Alkylcarbonyl, C₁-C₁₂Alkoxy-C₁-C₁₂-alkyl; C₁-C₁₂-Alkoxycarbonyl; C₁-C₁₂-Alkoxycarbonyl-C₁-C₁₂-alkyl; C₁-C₁₂-Halogenalkoxy-C₁-C₁₂-alkyl; Amino-C₁-C₁₂-alkyl, worin Amino gegebenenfalls mit 2 C₁-C₁₂-Alkylgruppen substituiert ist; gegebenenfalls substituierten Aryl-, Aryl-oxyl-, Aryl-C₁-C₁₂-alkyl-, Aryl-C₁-C₁₂-alkoxy-, Aryloxy-C₁-C₁₂-alkyl-, Aryl-C₁-C₁₂-alkoxy-C₁-C₁₂-alkyl-, Heteroaryl-, Heteroaryl-C₁-C₁₂-alkyl- und Heteroaryl-C₁-C₁₂-alkoxygruppen, wobei die optionalen Substituenten bis zu 3 Gruppen sind, die aus (a) Halogen, (b) NO₂, (c) C₁-C₆-Alkyl, (d) C₁-C₆-Halogenalkyl, (e) C₁-C₆-Alkoxy und (f) C₁-C₆-Alkoxy-C₁-C₆-alkyl ausgewählt sind; und Gruppen der Formel: 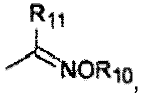 ausgewählt sind, worin R₁₀ und R₁₁ aus (a) H, (b) C₁-C₁₂-Alkyl, (c) Aryl und (d) Aryl-C₁-C₁₂-alkyl ausgewählt sind.

2. Benzopyron-Verbindung nach Anspruch 1, worin:
R₁ und R₂ jeweils unabhängig voneinander aus H, C₁-C₆-Alkyl und C₁-C₆-Halo-genalkyl ausgewählt sind;
R₃ aus H, C₁-C₆-Alkyl, C₁-C₆-Halogenalkyl und C₁-C₆-Alkoxy ausgewählt ist;
R₄, R₅, R₆, R₇ und R₈ gleich oder unterschiedlich sein können und aus H; Halogen; CN; NO₂; C₁-C₆-Alkyl; C₂-C₆-Alkenyl; C₂-C₆-Alkinyl, C₁-C₆-Halogenalkyl, C₁-C₆-Alkoxy; C₁-C₆-Alkylthio; C₁-C₆-Alkylsulfonyl; C₁-C₆-Alkylcarbonyl; C₁-C₆-Alkoxy-C₁-C₆-alkyl; C₁-C₆-Alkoxycarbonyl; C₁-C₆-Alkoxycarbonyl-C₁-C₆-alkyl; C₁-C₆-Halogen-alkoxy-C₁-C₆-alkyl; Amino-C₁-C₆-alkyl, worin Amino gegebenenfalls mit 2 C₁-C₁₂-Alkylgruppen substituiert ist; gegebenenfalls substituierten Aryl-, Aryloxyl-, Aryl-C₁-C₆-alkyl-, Aryl-C₁-C₆-alkoxy-, Aryloxy-C₁-C₆-alkyl-, Aryl-C₁-C₆-alkoxy-C₁-C₆-alkyl-, Heteroaryl-, Heteroaryl-C₁-C₆-alkyl- und Heteroaryl-C₁-C₆-alkoxygruppen, wobei die optionalen Substituenten bis zu 3 Gruppen sind, die aus (a) Halogen, (b) NO₂, (c) C₁-C₂-Alkyl, (d) C₁-C₂-Halogenalkyl, (e) C₁-C₂-Alkoxy und (f) C₁-C₂-Alkoxy-C₁-C₂-alkyl ausgewählt sind; und Gruppen der Formel: 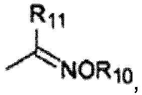 ausgewählt sind, worin R₁₀ und R₁₁ aus (a) H, (b) C₁-C₆-Alkyl, (c) Aryl und (d) Aryl-C₁-C₆-alkyl ausgewählt sind.

3. Benzopyron-Verbindung nach Anspruch 2, worin:
B O ist;
R₁ und R₂ beide Methyl sind;
R₃ H oder Methyl ist;
R₄, R₅, R₆, R₇ und R₈ gleich oder unterschiedlich sein können und aus H; Halogen; CN; NO₂; C₁-C₆-Alkyl; C₂-C₆-Alkenyl; C₁-C₆-Halogenalkyl, C₁-C₆-Alkoxy; C₁-C₆-Alkylcarbonyl; C₁-C₆-Alkoxy-C₁-C₆-alkyl; C₁-C₆-Alkoxycarbonyl; C₁-C₆-Alkoxycarbonyl-C₁-C₆-alkyl; C₁-C₃-Halogenalkoxy-C₁-C₃-alkyl; Amino-C₁-C₃-alkyl, worin Amino gegebenenfalls mit 2 C₁-C₃-Alkylgruppen substituiert ist; gegebenenfalls substituierten Phenyl-, Phenoxy-, Phenyl-C₁-C₂-alkyl-, Phenyl-C₁-C₂-alkoxy, Phenoxy-C₁-C₂-alkyl-, Phenylmethyl-, Phenylmethoxyl- und Phenylmethoxy-C₁-C₂-alkylgruppen, wobei die Substituenten bis zu 2 Gruppen sind, die aus (a) Halogen, (b) NO₂, (c) C₁-C₂-Alkyl, (d) C₁-C₂-Halogenalkyl, (e) C₁-C₂-Alkoxy und (f) C₁-C₂-Alkoxy-C₁-C₂-alkyl ausgewählt sind; und Gruppen der Formel: 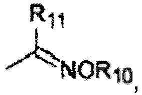 ausgewählt sind, worin R₁₀ und R₁₁ jeweils aus H und C₁-C₆-Alkyl ausgewählt sind.

4. Benzoypron-Verbindung nach Anspruch 3, worin:
R₄, R₅, R₆, R₇ und R₈ gleich oder unterschiedlich sein können und aus H; Cl; Br; F; CN; C₁-C₆-Alkyl; C₁-C₆-Halogenalkyl; C₁-C₆-Alkylcarbonyl; C₁-C₆-Alkoxy; C₁-C₆-Alkoxy-C₁-C₃-alkyl; C₁-C₃-Halogenalkoxy-C₁-C₃-alkyl; Amino-C₁-C₃-alkyl, worin Amino gegebenenfalls mit bis zu 2 C₁-C₃-Alkylgruppen substituiert ist; gegebenenfalls substituierten Phenyl-, Phenoxy-, Phenylmethyl- und Phenylmethoxylgruppen, wobei die Substituenten bis zu 2 Gruppen sind, die aus (a) Halogen, (b) NO₂, (c) C₁-C₂-Alkyl, (d) C₁-C₂-Halogenalkyl, (e) C₁-C₂-Alkoxy und (f) C₁-C₂-Alkoxy-C₁-C₂-alkyl ausgewählt sind; und Gruppen der Formel: 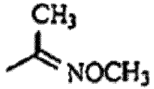 ausgewählt sind.

5. Herstellungsverfahren für eine Benzopyronverbindung nach einem der vorangegangenen Ansprüche, wobei die Verbindung der allgemeinen Formel (I) durch Umsetzen von Benzylhalogenid der allgemeinen Formel (II) mit einer 7-OH-Benzopyron-Verbindung der allgemeinen Formel (III) in Gegenwart einer Base hergestellt wird: 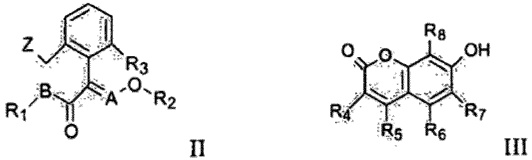 worin:
Z eine Abgangsgruppe ist, die aus Cl oder Br ausgewählt ist; und A, B und R₁ bis R₈ wie in einem vorangegangenen Anspruch definiert sind.

6. Verwendung einer Benzopyron-Verbindung nach einem der Ansprüche 1 bis 4 zur Bekämpfung von Insekten in Pflanzen.

7. Verwendung einer Benzopyron-Verbindung nach einem der Ansprüche 1 bis 4 zur Bekämpfung von Pilzen in Pflanzen.

8. Fungizid- oder Insektizidzusammensetzung, die eine Verbindung nach Anspruch 1 als Wirkbestandteil umfasst, wobei der gewichtsprozentuelle Gehalt des Wirkbestandteils in der Zusammensetzung 0,1 % bis 99 % beträgt.

## Revendications

1. Composé benzopyrone de formule générale (I) : 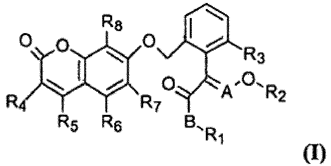 ou un stéréoisomère de celui-ci, dans lequel :
A est CH ou N ;
B est 0 ou S ;
R₁ et R₂ sont respectivement choisis parmi H, un groupe alkyle en C₁ à C₁₂ ou un groupe halogénoalkyle en C₁ à C₁₂ ;
R₃ est choisiparmi H, un groupe alkyle en C₁ à C₁₂, un groupe halogénoalkyle en C₁ à C₁₂ ou un groupe alcoxy en C₁ à C₁₂ ;
R₄, R₅, R₆, R₇ et R₈ peuvent être identiques ou différents et sont choisis parmi : H, un atome d'halogène ; CN ; NO₂ ; un groupe alkyle en C₁ à C₁₂ ; un groupe alcényle en C₂ à C₁₂ ; un groupe alcynyle en C₂ à C₁₂ ; un groupe halogénoalkyle en C₁ à C₁₂ ; un groupe alcoxy en C₁ à C₁₂ ; un groupe alkylthio en C₁ à C₁₂ ; un groupe alkylsulfonyle en C₁ à C₁₂ ; un groupe alkylcarbonyle en C₁ à C₁₂ ; un groupe (alcoxy en C₁ à C₁₂) alkyle en C₁ à C₁₂ ; un groupe alcoxycarbonyle en C₁ à C₁₂ ; ;un groupe (alcoxycarbonyle en C₁ à C₁₂) alkyle en C₁ à C₁₂ ; un groupe (halogénoalcoxy en C₁ à C₁₂) alkyle en C₁ à C₁₂ ; un groupe aminoalkyle en C₁ à C₁₂ dans lequel le groupe amino est facultativement substitué par jusqu'à deux groupes alkyle en C₁ à C₁₂ ; aryle facultativement substitué, aryloxyle, arylalkyle en C₁ à C₁₂, arylalcoxy en C₁ à C₁₂, aryloxyalkyle en C₁ à C₁₂, (arylalcoxy en C₁ à C₁₂) alkyle en C₁ à C₁₂, hétéroaryle, hétéroarylalkyle en C₁ à C₁₂ ou hétéroarylalcoxy en C₁ à C₁₂, lesdits substituants facultatifs étant jusqu'à 3 groupes choisis parmi (a) un atome d'halogène, (b) NO₂, (c) un groupe alkyle en C₁ à C₆, (d) un groupe halogénoalkyle en C₁ à C₆, (e) un groupe alcoxy en C₁ à C₆ et (f) un groupe (alcoxy en C₁ à C₆) alkyle en C₁ à C₆ ; et les groupes de formule : 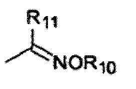 dans laquelle R₁₀ et R₁₁ sont choisis parmi (a) H, (b) un groupe alkyle en C₁ à C₁₂, (c) un groupe aryle et (d) un groupe arylalkyle en C₁ à C₁₂.

2. Composé benzopyrone selon la revendication 1, dans lequel :
R₁ et R₂ sont respectivement choisis parmi H, un groupe alkyle en C₁ à C₆ ou un groupe halogénoalkyle en C₁ à C₆ ;
R₃ est choisiparmi H, un groupe alkyle en C₁ à C₆, un groupe halogénoalkyle en C₁ à C₆ ou un groupe alcoxy en C₁ à C₆ ;
R₄, R₅, R₆, R₇ et R₈ peuvent être identiques ou différents et sont choisis parmi : H, un atome d'halogène ; CN ; NO₂ ; un groupe alkyle en C₁ à C₆ ; un groupe alcényle en C₂ à C₆ ; un groupe alcynyle en C₂ à C₆ ; un groupe halogénoalkyle en C₁ à C₆ ; un groupe alcoxy en C₁ à C₆ ; un groupe alkylthio en C₁ à C₆ ; un groupe alkylsulfonyle en C₁ à C₆ ; un groupe alkylcarbonyle en C₁ à C₆ ; un groupe (alcoxy en C₁ à C₆ alkyle en C₁ à C₆ ; un groupe alcoxycarbonyle en C₁ à C₆ ;un groupe (alcoxycarbonyle en C₁ à C₆) alkyle en C₁ à C₆ ; un groupe (halogénoalcoxy en C₁ à C₆) alkyle en C₁ à C₆ ; un groupe aminoalkyle en C₁ à C₆ dans lequel le groupe amino est facultativement substitué par jusqu'à deux groupes alkyle en C₁ à C₁₂ ; aryle facultativement substitué, aryloxyle, arylalkyle en C₁ à C₆, arylalcoxy en C₁ à C₆, aryloxyalkyle en C₁ à C₆, (arylalcoxy en C₁ à C₆) alkyle en C₁ à C₆, hétéroaryle, hétéroarylalkyle en C₁ à C₆ ou hétéroarylalcoxy en C₁ à C₆, lesdits substituants facultatifs étant jusqu'à 3 groupes choisis parmi (a) un atome d'halogène, (b) NO₂, (c) un groupe alkyle en C₁ à C₂, (d) un groupe halogénoalkyle en C₁ à C₂, (e) un groupe alcoxy en C₁ à C₂ et (f) un groupe (alcoxy en C₁ à C₂) alkyle en C₁ à C₂ ; et les groupes de formule : 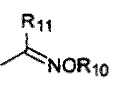 dans laquelle R₁₀ et R₁₁ sont choisis parmi (a) H, (b) un groupe alkyle en C₁ à C₆, (c) un groupe aryle et (d) un groupe arylalkyle en C₁ à C₆.

3. Composé benzopyrone selon la revendication 2, dans lequel :
B est O ;
R₁ et R₂ sont tous deux des groupes méthyle ;
R₃ est H ou un groupe méthyle ;
R₄, R₅, R₆, R₇ et R₈ peuvent être identiques ou différents et sont choisis parmi H, un atome d'halogène, CN, NO₂, un groupe alkyle en C₁ à C₆, un groupe alcényle en C₂ à C₆, un groupe halogénoalkyle en C₁ à C₆, un groupe alcoxy en C₁ à C₆, un groupe alkylcarbonyle en C₁ à C₆, un groupe (alcoxy en C₁ à C₆) alkyle en C₁ à C₆, un groupe alcoxycarbonyle en C₁ à C₆, un groupe (alcoxycarbonyle en C₁ à C₆) alkyle en C₁ à C₃, un groupe (halogénoalcoxy en C₁ à C₃) alkyle en C₁ à C₃, un groupe aminoalkyle en C₁ à C₃ dans lequel le groupe amino est facultativement substitué par jusqu'à deux groupes alkyle en C₁ à C₃ ; phényle facultativement substitué, phénoxy, phénylalkyle en C₁ à C₂, phénylalcoxy en C₁ à C₂, phénoxyalkyle en C₁ à C₂, phénylméthyle, phénylméthoxy ou phénylméthoxyalkyle en C₁ à C₂, lesdits substituants étant jusqu'à 2 groupes choisis parmi : (a) un atome d'halogène, (b) NO₂, (c) un groupe alkyle en C₁ à C₂, (d) un groupe halogénoalkyle en C₁ à C₂, (e) un groupe alcoxy en C₁ à C₂ et (f) un groupe (alcoxy en C₁ à C₂) alkyle en C₁ à C₂ ; et les groupes de formule : 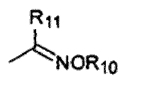 dans laquelle R₁₀ et R₁₁ sont choisis parmi H et un groupe alkyle en C₁ à C₆.

4. Composé benzopyrone selon la revendication 3, dans lequel :
R₄, R₅, R₆, R₇ et R₈ peuvent être identiques ou différents et sont choisis parmi H ; Cl ; Br ; F ; CN ; un groupe alkyle en C₁ à C₆ ; un groupe halogénoalkyle en C₁ à C₆ ; un groupe alkylcarbonyle en C₁ à C₆ ; un groupe alcoxy en C₁ à C₆ ; un groupe (alcoxy en C₁ à C₆) alkyle en C₁ à C₃ ; un groupe (halogénoalcoxy en C₁ à C₃) alkyle en C₁ à C₃ ; un groupe aminoalkyle en C₁ à C₃ dans lequel le groupe amino est facultativement substitué par jusqu'à deux groupes alkyle en C₁ à C₃ ; phényle facultativement substitué, phénoxy, phénylméthyle ouphénylméthoxy, lesdits substituants étant jusqu'à 2 groupes choisis parmi : (a) un atome d'halogène, (b) NO₂, (c) un groupe alkyle en C₁ à C₂, (d) un groupe halogénoalkyle en C₁ à C₂, (e) un groupe alcoxy en C₁ à C₂ et (f) un groupe (alcoxy en C₁ à C₂) alkyle en C₁ à C₂ ; et les groupes de formule :

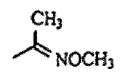

5. Procédé de préparation d'un composé benzopyrone selon l'une quelconque des revendications précédentes, dans lequel le composé de formule générale (I) est préparé en faisant réagir un halogénure de benzyle répondant à la formule générale (II) avec un composé 7-OH-benzopyrone répondant à la formule générale (III) en présence d'une base : 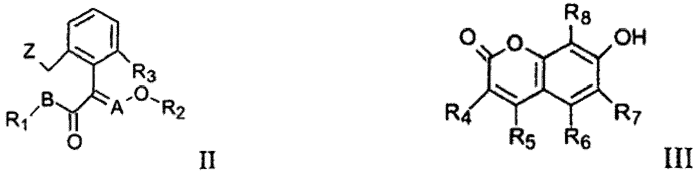 dans lesquelles :
Z est un groupe partant choisi parmi Cl ou Br ; et A, B et R₁ à R₈ sont tels que définis selon l'une quelconque des revendications précédentes.

6. Utilisation d'un composé benzpyrone selon l'une quelconque des revendications 1 à 4 pour lutter contre les insectes dans les plantes.

7. Utilisation d'un composé benzpyrone selon l'une quelconque des revendications 1 à 4 pour lutter contre les champignons dans les plantes.

8. Composition fongicide et insecticide qui comprend le composé selon la revendication 1 en tant que principe actif, dans laquelle le pourcentage en poids du principe actif dans la composition est de 0,1 % à 99 %.
